# EUROPÄISCHE PATENTSCHRIFT

(11) **EP 2 143 393 B1**
(45) Veröffentlichungstag und Bekanntmachung des Hinweises auf die Patenterteilung: **08.06.2016**
(21) Anmeldenummer: 09164793.3
(22) Anmeldetag: 07.07.2009
(51) Int. Cl.: A61B 17/34, A61B 17/00

(54) **Chirurgische Schutzvorrichtung für ein chirurgisches Dichtelement und chirurgisches Abdichtungssystem**
Surgical protection device for a surgical sealing element in a surgical sealing system
Dispositif de protection chirurgical pour un élément d'étanchéité chirurgical et système d'étanchéification chirurgical

(30) Priorität: 09.07.2008 DE 102008033374
(43) Veröffentlichungstag der Anmeldung: 13.01.2010
(73) Patentinhaber: Aesculap AG, 78532 Tuttlingen (DE)
(72) Erfinder: Schweitzer, Tom, 78532, Tuttlingen (DE); Mayenberger, Rupert, 78239, Rielasingen (DE)
(74) Vertreter: Hoeger, Stellrecht & Partner Patentanwälte mbB

(56) Entgegenhaltungen:
- EP-A- 1 671 596
- WO-A-01/89397
- WO-A-93/01850
- WO-A-2007/110371
- WO-A-2007/121425
- US-A- 5 342 315
- US-A- 5 662 615
- US-A1- 2006 211 992

## Beschreibung

Die vorliegende Erfindung betrifft eine chirurgische Schutzvorrichtung für ein chirurgisches, eine erweiterbare Einführöffnung aufweisendes Dichtelement eines chirurgischen, einen Trokar umfassenden Abdichtungssystems, welche Schutzvorrichtung einen am Trokar oder an einem Teil desselben anordenbaren, ringförmigen oder im Wesentlichen ringförmigen und eine Durchbrechung definierenden Grundkörper mit mehreren in Umfangsrichtung angeordneten und parallel oder auf eine Längsachse der Schutzvorrichtung hin weisenden Schutzelementen umfasst, welche Schutzelemente im Wesentlichen in distaler Richtung weisende freie Enden aufweisen, wobei mindestens ein Teil der Schutzelemente auf einer Außenseite mindestens ein Rückhalteelement zum in Eingriff Bringen mit dem Dichtelement aufweisen.

Ferner betrifft die vorliegende Erfindung ein chirurgisches Abdichtungssystem umfassend einen Trokar mit einer Trokarhülse, ein an der Trokarhülse gehaltenes chirurgisches, eine erweiterbare Einführöffnung aufweisendes Dichtelement zum Abdichten der Einführöffnung beim Einführen eines chirurgischen Instruments und eine chirurgische Schutzvorrichtung für das Dichtelement, welche Schutzvorrichtung einen am Trokar oder am Dichtelement anordenbaren, ringförmigen oder im Wesentlichen ringförmigen und eine Durchbrechung definierenden Grundkörper mit mehreren in Umfangsrichtung angeordneten und parallel oder auf eine Längsachse der Schutzvorrichtung hin weisenden Schutzelementen umfasst, welche Schutzelemente im Wesentlichen in distaler Richtung weisende freie Enden aufweisen, wobei mindestens ein Teil der Schutzelemente auf einer Außenseite mindestens ein Rückhalteelement zum in Eingriff Bringen mit dem Dichtelement aufweisen.

Eine Schutzvorrichtung sowie ein Abdichtungssystem der eingangs beschriebenen Art, deren Schutzelemente auf einer Außenseite jedoch kein Rückhalteelement zum in Eingriff Bringen mit dem Dichtelement aufweisen, sind beispielsweise aus der EP 0 696 459 B1 bekannt. Die Schutzvorrichtung dient dazu, das Dichtelement, welches häufig aus einem elastischen Material hergestellt ist, beim Einführen von Instrumenten mit spitzen Enden in den Trokar zu schützen. Spitze Enden von Instrumenten, beispielsweise Spitzen und Kanten an Werkzeugelementen der Instrumente, können dazu führen, dass sich die Instrumentenspitze im Dichtungselement verhakt und somit das Einführen des Instruments erschwert oder gar unmöglich wird. Im ungünstigsten Fall kann das Dichtelement dabei sogar zerstört werden. Des Weiteren haben die Schutzelemente den Vorteil, dass sie eine Aufdehnung der Einführöffnung bei Instrumenten mit großen Durchmessern unterstützen. Die bekannten, lamellenartigen Schutzelemente liegen üblicherweise auf dem Dichtelement auf, so dass es insbesondere beim Einführen eines Instruments durch die Einführöffnung des Dichtelements zu einer nachteiligen Relativbewegung zwischen dem Schutzelement und dem Dichtelement kommen kann.

Eine Trokardichtung sowie eine Schutzvorrichtung für diese ist aus der US 5,342,315 bekannt. Die US 5,342,315 offenbart eine chirurgishe Schutzvorrichtung und ein chirurgisches Abdichtungs system entsprechend dem Oberbegriff des Anspruchs 1 und des Anspruchs 9. Die WO 93/01850 A1 offenbart eine Hebel betätigbare Septumdichtung. In der WO 01/89397 A1 ist eine chirurgische Dichtung beschrieben. Aus der WO 2007/121425 A1 ist eine weitere Trokardichtung bekannt. Die WO 2007/110371 A1 offenbart ein chirurgisches Dichtelement, eine chirurgische Dichtung und ein chirurgisches Abdichtungssystem. Ein weiteres Abdichtungssystem mit einer Trokardichtung ist in der US 2006/0211992 A1 beschrieben.

Nachteilig bei den bekannten Schutzvorrichtungen ist es, dass die Schutzelemente nicht immer genau bis an den Dichtbereich heranreichen. Daher kann es vorkommen, dass ungeschützte Bereiche des Dichtelements verbleiben, die von spitzen Instrumentenenden verletzt werden können.

Es ist daher eine Aufgabe der Erfindung, eine chirurgische Schutzvorrichtung sowie ein chirurgisches Abdichtungssystem der eingangs beschriebenen Art so zu verbessern, dass unabhängig von einem Aufdehnungsgrad des Dichtelements ein möglichst vollständiger Schutz desselben erreicht werden kann.

Diese Aufgabe wird bei einer chirurgischen Schutzvorrichtung sowie einem chirurgischen Abdichtungssystem der eingangs beschriebenen Art erfindungsgemäß dadurch gelöst, dass das mindestens eine Rückhalteelement an einem freien Ende oder im Bereich eines freien Endes eines Schutzelements angeordnet ist und dass es eine vom Schutzelement weg weisende Spitze und eine Form aufweist, welche ein Verhaken mit einem elastischen Dichtelement ermöglicht.

Die vorgeschlagene Weiterbildung der bekannten Schutzvorrichtung sowie des bekannten Abdichtungssystems hat insbesondere den Vorteil, dass es gezielt zu einem Verhaken der Schutzvorrichtung mit dem Dichtelement kommen kann, das beim Einführen der Instrumente gerade vermieden werden soll. Beim Einführen von Instrumenten hat das in Eingriff Bringen der Rückhalteelemente mit dem Dichtelement den Vorteil, dass so sichergestellt ist, dass ein Schutz des Dichtelements bis an den Bereich des Dichtelements, welcher am eingeführten Instrumentenschaft anliegt, also insbesondere eine definierte Dichtlinie oder Dichtlippe, sichergestellt werden kann. Durch das in Eingriff Bringen der Rückhalteelemente mit dem Dichtelement führt eine Aufweitung der Einführöffnung der Schutzvorrichtung automatisch auch zu einer Aufdehnung des Dichtelements. Eine Relativbewegung der Schutzvorrichtung und des Dichtelements wird jedoch im Wesentlichen verhindert. Dies hat den Vorzug, dass eine ab dem in Eingriff Bringen der Rückhalteelemente mit dem Dichtelement vorgegebene Bedeckung desselben in axialer Richtung der Schutzvorrichtung unabhängig von einer Einführstellung des eingeführten Instruments sichergestellt werden kann. Ein in Eingriff Bringen kann insbesondere dann erfolgen, wenn das Rückhalteelement einen minimalen Durchmesser aufweist, so dass es dann, wenn es auf beispielsweise ein aus einem Elastomer hergestelltes Dichtelement trifft, dessen Wand ausbeult und so quasi eine korrespondierende Ausnehmung definiert, in welche das Rückhalteelement eingreift. Durch das Ausbeulen wird jedoch ein Entlanggleiten des das Dichtelement ausbeulenden Gegenstands am Dichtelement verhindert. Vorzugsweise ist an den jeweiligen Schutzelementen ein einziges Rückhalteelement vorgesehen. Prinzipiell wäre es auch denkbar, mehrere Rückhalteelemente, also zwei, drei oder mehr an jeweils einem Schutzelement vorzusehen, um durch in Eingriff Bringen der Rückhalteelemente mit dem Dichtelement eine Relativbewegung der beiden Teile beim Auffalten des Dichtelements zum Aufweiten der Einführöffnung zu verhindern. Damit eine Wand des Dichtelements auf einfache Weise zum in Eingriff Bringen ausgebeult werden kann, ist es günstig, dass die Rückhalteelemente eine vom Schutzelement weg weisende Spitze aufweisen. Die Rückhalteelemente weisen ferner eine Form auf, welche ein Verhaken mit einem elastischen Dichtelement ermöglicht.

Günstig ist es, wenn das mindestens eine Rückhalteelement in Form eines vom Schutzelement abstehenden Rückhaltevorsprungs ausgebildet ist. Derartige Rückhalteelemente sind besonders einfach herzustellen und können entsprechend dimensioniert werden, um ein gezieltes Verhaken oder anderweitiges in Eingriff Bringen der Rückhaltevorsprünge mit dem Dichtelement sicherzustellen.

Je nach Orientierung der Schutzelemente in einer Grundstellung oder auch einer aufgeweiteten Stellung, ist es vorteilhaft, wenn mindestens ein Teil der Rückhaltevorsprünge senkrecht oder im Wesentlichen senkrecht von den Schutzelementen absteht. Zudem ist eine derartige Ausgestaltung der Rückhaltevorsprünge besonders einfach herzustellen.

Ein Verhaken oder in Eingriff Bringen der Rückhaltevorsprünge mit dem Dichtelement kann insbesondere dann, wenn die Schutzelemente parallel zu einer vom Dichtelement definierten Längsachse am Grundkörper abstehen, verbessert werden, wenn mindestens ein Teil der Rückhaltevorsprünge schräg bezogen auf eine Erstreckung der Schutzelemente im Bereich ihrer freien Enden von diesen weg weisend absteht. Insbesondere weisen derartige Rückhaltevorsprünge vom Grundkörper weg und nach außen in Richtung auf das Dichtelement hin. So kann einfach und sicher ein Verhaken der Rückhaltevorsprünge mit dem Dichtelement sichergestellt werden.

Vorzugsweise beträgt ein Durchmesser der Rückhalteelemente maximal 1 mm. Je nach Größe sowie abhängig von einer Wandstärke des Dichtelements kann es jedoch auch vorteilhaft sein, wenn ein Durchmesser der Rückhalteelemente maximal 0,5 mm beträgt. Günstigerweise beträgt ein Durchmesser der Rückhalteelemente maximal 0,2 mm.

Um das Dichtelement mit dem Rückhalteelement nicht zu verletzen, ist es vorteilhaft, wenn die Spitze kegelförmig oder kugelig geformt ist oder wenn es ein kugeliges oder kegelförmiges freies Ende aufweist. Diese Ausgestaltungen ermöglichen eine Ausbeulung in gewünschter Weise, verhindern jedoch ein Durchstechen des Dichtelements. Insbesondere beträgt eine Höhe der Rückhalteelemente bezogen auf das jeweilige Schutzelement maximal 1,5 mm. Vorzugsweise weist die Höhe der Rückhalteelemente einen Wert im Bereich von etwa 0,3 mm bis etwa 1,0 mm auf.

Besonders sicher in Eingriff bringbar mit dem Dichtelements ist ein Rückhalteelement, welches eine Spitze mit einem Radius in einem Bereich von etwa 0,1 mm bis etwa 0,6 mm aufweist.

Damit eine Relativposition zwischen den Schutzelementen und dem Dichtelement unabhängig von einer Öffnungsstellung des Dichtelements gleich bleibt, ist es günstig, wenn die Rückhalteelemente aus einem Material mit einem hohen Haftreibungskoeffizienten hergestellt sind. Insbesondere wäre es auch denkbar, am Schutzelement nur einen Flächenbereich mit einem entsprechend hohen Haftreibungskoeffizienten vorzusehen, so dass das Dichtelement auch bei im Wesentlichen parallel zu dessen Oberfläche wirkenden Kräften am Schutzelement haften bleibt und so keine Relativbewegung zwischen dem Schutzelement und dem Dichtelement möglich ist.

Vorteilhaft ist es, wenn die Rückhalteelemente in Form einer Antigleitbeschichtung ausgebildet oder mit einer Antigleitbeschichtung versehen sind. Die Antigleitbeschichtung verhindert eine Relativbewegung der Rückhalteelemente beziehungsweise des Schutzelements relativ zum Dichtelement, da sie ein Gleiten relativ zum Dichtelement verhindert.

Gemäß einer bevorzugten Ausführungsform der Erfindung kann eine am Grundkörper angeordnete Verbindungseinrichtung zum Verbinden der Schutzvorrichtung mit dem chirurgischen Dichtelement oder dem chirurgischen Abdichtungssystem vorgesehen sein. Die Verbindungseinrichtung kann insbesondere auch die Funktion einer Verdrehsicherung zum Verhindern einer Rotation der Schutzvorrichtung um dessen Längsachse übernehmen, insbesondere wenn sie formschlüssig mit dem chirurgischen Dichtelement beziehungsweise mit dem chirurgischen Abdichtungssystem in Eingriff gebracht werden kann. Ferner erlaubt eine solche Ausgestaltung eine definierte Positionierung der Schutzeinrichtung relativ zum Dichtelement.

Auf besonders einfache Weise lässt sich die Verbindungseinrichtung ausbilden, wenn sie mindestens ein in radialer Richtung vom Grundkörper abstehendes Verbindungselement umfasst. Beispielsweise kann es sich bei dem Verbindungselement um einen vom Grundkörper mindestens teilweise in radialer Richtung abstehenden, umlaufenden Ringflansch oder um eine umlaufende Ringnut handeln.

Vorzugsweise sind mehrere, in Umfangsrichtung voneinander beabstandete Verbindungselemente vorgesehen. Derartige Verbindungselemente am Grundkörper anzuordnen, ermöglicht eine definierte Positionierung der Schutzvorrichtung am Dichtelement oder an einem anderen Teil des Abdichtungssystems. Die Verbindungselemente können in Form von mindestens teilweise in radialer Richtung abstehenden Vorsprüngen und/oder Ausnehmungen am Grundkörper ausgebildet sein. Für eine einfache Verbindung der Verbindungselemente mit dem Dichtelement oder einem anderen Teil des Abdichtungssystems können an diesen korrespondierende Elemente ausgebildet sein. Selbstverständlich können auch zwei oder mehr Verbindungselemente parallel zueinander und in axialer Richtung versetzt in Umfangsrichtung am Grundkörper angeordnet sein. Eines der Verbindungselemente kann beispielsweise in Form eines umlaufenden Ringflansches eine Positionierung in axialer Richtung sichern, zwei oder mehr hierzu in axialer Richtung versetzte und in Umfangsrichtung voneinander beabstandete Verbindungselemente eine Orientierung um eine Längsachse der Schutzvorrichtung vorgeben und somit auch eine Verdrehsicherung definieren.

Besonders einfach in der Herstellung wird die chirurgische Schutzvorrichtung, wenn die Verbindungselemente gleichmäßig über den Umfang des Grundkörpers verteilt angeordnet sind. Des Weiteren wird so auch eine Montage besonders einfach, da die Schutzvorrichtung nur in definierten Positionen entsprechend der Zahl der vorgesehenen Verbindungselemente relativ zum Dichtungselement oder zum Abdichtungssystem angeordnet und positioniert werden kann.

Um eine möglichst gleichmäßige Verformung der Schutzelemente infolge des Einführens eines Instruments zu erreichen, ist es günstig, wenn die Schutzelemente eine längs ihrer Erstreckung konstante Dicke aufweisen.

Vorzugsweise sind in einer Grundstellung der Schutzvorrichtung die freien Enden der Schutzelemente nähe der Längsachse angeordnet. Dadurch kann sichergestellt werden, dass bereits beim Einführen von Instrumenten mit sehr kleinen Schaftdurchmessern die Instrumente in jedem Fall zunächst Innenflächen der Schutzelemente berührt, um die Schutzelemente dann in radialer Richtung nach außen zu bewegen, wodurch ein in Eingriff Bringen der Rückhalteelemente mit dem Dichtungselement erfolgen kann. Die Anordnung der freien Enden der Schutzelemente nahe der Längsachse kann insbesondere dadurch erreicht werden, dass die Schutzelemente ausgehend vom Grundkörper weg nach innen gewölbt sind.

Um sicherzustellen, dass die Rückhalteelemente sofort nach dem Einführen eines Instruments in Kontakt mit dem Dichtelement kommen können, ist es günstig, wenn die freien Enden der Schutzelemente etwas von der Längsachse weg weisend gekrümmt sind. Vorzugsweise weisen die freien Enden in der Grundstellung etwas von der Längsachse weg, so dass insbesondere ein freies Ende der Rückhalteelemente einen größeren Abstand von der Längsachse aufweisen kann als ein Innenwandabschnitt des jeweiligen Schutzelements von der Längsachse. Dadurch können beispielsweise infolge eines in Kontakt Kommens eines Instruments mit den Schutzelementen beim Einführen des Instrments die freien Enden in radialer Richtung nach außen gedrängt werden, so dass die Rückhalteelemente mit dem Dichtungselement in einfacher Weise sicher in Eingriff kommen können.

Vorteilhaft ist es, wenn sich in einer Grundstellung der Schutzvorrichtung benachbarte Schutzelemente gegenseitig teilweise überdecken. Insbesondere können sie sich in Richtung auf ihre freien Enden hin zunehmend überdecken oder überlappen. Dies stellt sicher, dass bei einem Auffalten des Dichtelements verbunden mit einem Aufweiten der Schutzvorrichtung, also insbesondere einer Bewegung der freien Enden der Schutzelemente von der Längsachse weg, stets eine ausreichende Bedeckung des Dichtelements mit den Schutzelementen sichergestellt werden kann, um eine Verletzung des Dichtelements durch in das Abdichtungssystem eingeführte Instrumente zu verhindern.

Gemäß einer bevorzugten Ausführungsform der Erfindung kann vorgesehen sein, dass in einer Grundstellung der Schutzvorrichtung die Rückhalteelemente tragenden Schutzelemente die Schutzelemente ohne Rückhalteelemente außen mindestens teilweise überdecken. Auf diese Weise kann sichergestellt werden, dass zunächst die Schutzelemente mit ihren Rückhalteelementen mit dem Dichtungselement in Kontakt kommen können.

Um einen möglichst kompakten Aufbau der Schutzvorrichtung, insbesondere in einer Grundstellung, zu ermöglichen, in welcher eine von den Schutzelementen begrenzte Einführöffnung möglichst klein ist, ist es vorteilhaft, wenn abwechselnd Schutzelemente mit und ohne Rückhalteelemente am Grundkörper angeordnet sind. Dies vereinfacht die Herstellung und macht nur eine minimale Anzahl von Rückhalteelementen erforderlich, um die gewünschte Wirkung der Schutzvorrichtung zu erreichen.

Vorteilhafterweise nimmt eine Breite der Rückhalteelemente tragenden Schutzelemente im Bereich der freien Enden ab. Dies ermöglicht es insbesondere, die freien Enden der Schutzelemente in entsprechende Konturen des Dichtelements einzupassen, beispielsweise in entsprechende Wellenberge oder Wellentäler einer die Längsachse wellenförmig umgebenden Dichtlinie oder Dichtlippe des Dichtungselements. So wird sichergestellt, dass die freien Enden der Schutzelemente bis an die Dichtlinie oder Dichtlippe des Dichtungselements heranreichen und durch das in Eingriff Bringen der Rückhalteelemente diese Positionierung auch unabhängig von einer Aufweitung des Dichtelements erfolgen kann.

Um auf einfache Weise ein Aufweiten einer von den Schutzelementen der Schutzvorrichtung definierten Einführöffnung zu ermöglichen, ist es günstig, wenn die Schutzelemente verschwenkbar am Grundkörper angeordnet sind. Dies kann beispielsweise mittels einer Schwenklagerung erfolgen oder auch durch ein Film- oder Scharniergelenk, über welches die Schutzelemente mit dem Grundkörper verbunden sind. Alternativ wäre eine Verschwenkbarkeit aber auch durch eine flexible oder teilweise elastische Ausgestaltung der Schutzelemente erreichbar.

Besonders vorteilhaft ist es, wenn die Schutzelemente in sich flexibel sind. Dies ermöglicht auch eine Bewegung der Schutzelemente von einer Längsachse der Schutzvorrichtung weg nach außen oder auch wieder zurück auf diese hin. Eine Flexibilität der Schutzelemente ist vorzugsweise derart vorgesehen, dass es nicht zu einem Abknicken der Schutzelemente infolge eingeführter Instrumente kommen kann.

Grundsätzlich wäre es denkbar, die Schutzvorrichtung zwei- oder mehrteilig auszubilden. Vorzugsweise ist sie jedoch einstückig ausgebildet. Dies ermöglicht es, die Schutzvorrichtung in einem einzigen Herstellungsschritt herzustellen, wodurch diese einfach und kostengünstig wird.

Günstigerweise ist die Schutzvorrichtung aus einem sterilisierbaren Material hergestellt. Vorzugsweise ist das Material dampf- und/oder gamma-sterilisierbar.

Besonders kostengünstig herstellen lässt sich die Schutzvorrichtung, wenn sie aus einem Kunststoff hergestellt ist. Insbesondere kann sie dann aus einem Teil durch Spritzgießen hergestellt werden.

Vorzugsweise umfasst das chirurgische Abdichtungssystem eine der oben beschriebenen Schutzvorrichtungen. Mit einer solchen Schutzvorrichtung verbessert sich die Handhabung und Bedienung auch des Abdichtungssystems in der jeweils beschriebenen Weise.

Gemäß einer bevorzugten Ausführungsform der Erfindung kann vorgesehen sein, dass das Dichtelement zum Abdichten von Schäften langgestreckter chirurgischer Instrumente beim Einführen in einen menschlichen oder tierischen Körper ausgebildet ist, eine Längsachse definiert und eine im Durchmesser veränderliche und quer oder im Wesentlichen quer zur Längsachse orientierte Öffnung aufweist, durch die ein Schaft einführbar ist. In einer Grundstellung ist das Dichtelement vorzugsweise so ausgebildet, dass es einen minimalen Durchmesser der Öffnung definiert. Beim Einführen eines Instruments kann das Dichtelement vorzugsweise aufgeweitet werden, so dass sich dessen Öffnung an einen Durchmesser des Schafts anpasst und diesen vollständig abdichtet, so dass keine Fluide, weder Gase noch Flüssigkeiten, zwischen dem Schaft und dem Dichtelement durchtreten können.

Günstig ist es, wenn das Dichtelement eine ringförmig geschlossene, flexible Wand umfasst, wenn die Wand einen ersten und einen zweiten, jeweils in sich geschlossenen Rand aufweist und wenn der erste Rand die Öffnung begrenzt. Der erste Rand kann insbesondere eine Dichtlinie oder eine Dichtlippe definieren, ausbilden oder tragen, so dass eine optimale Abdichtung eines eingeführten Instrumentenschafts, insbesondere um einen Gasverlust beim Einsatz des Abdichtungssystems bei einer minimalinvasiven Bauchoperation zu vermeiden, erreicht werden kann.

Eine besonders gute Abdichtung kann erreicht werden, wenn das Dichtelement eine ringförmig geschlossene, flexible Wand umfasst, wenn die Wand einen ersten und einen zweiten, jeweils in sich geschlossenen Rand aufweist und wenn der erste Rand die Öffnung begrenzt.

Günstigerweise ist die Wand wellenförmig faltbar und in einer Dichtstellung derart wellenförmig ohne Knicke mit in Richtung auf den ersten Rand hin verlaufenden Faltlinien gefaltet derart, dass der erste Rand eine Wellenlinie definiert, welche vollständig auf einer Zylinderfläche liegt. Der erste Rand ermöglicht es insbesondere, im Querschnitt kreisförmige Instrumente perfekt abzudichten. Der erste, eine Wellenlinie definierende Rand umgibt einen Instrumentenschaft, wobei er, anders als bei herkömmlichen Dichtelementen, keine kreisringförmige Anlage am Instrumentenschaft definiert, sondern eine in sich geschlossene ringförmige, in einer Seitenansicht wellenförmige Dichtlinie. Der erste Rand kann insbesondere auch eine Dichtlippe tragen oder anderweitig wulstförmig ausgebildet sein, um eine besonders gute und definierte Abdichtung relativ zu einem eingeführten Instrumentenschaft zu erreichen.

Um die Dichteigenschaften weiter zu verbessern, ist es vorteilhaft, wenn die Zylinderfläche konzentrisch zur Längsachse orientiert ist.

Damit bereits eine optimale Abdichtung von Schäften mit minimalen Durchmessern sichergestellt werden kann, ist es günstig, wenn das Dichtelement in einer Grundstellung eine Dichtungsstellung einnimmt, in der die Öffnung einen minimalen Durchmesser aufweist.

Vorteilhaft ist es, wenn das Dichtelement in der Grundstellung eine in Richtung auf den ersten Rand hin wellenförmig ohne Knicke gefaltete Wand aufweist und wenn der erste Rand eine Wellenlinie definiert, welche vollständig auf einer Zylinderfläche liegt. So kann direkt nach dem Einführen eines Instrumentenschafts dieser perfekt durch das Dichtelement abgedichtet werden.

Gemäß einer bevorzugten Ausführungsform der Erfindung kann vorgesehen sein, dass die Wellenlinie oberhalb einer senkrecht zur Längsachse verlaufenden Öffnungsebene der Öffnung Wellenberge und unterhalb der Öffnungsebene Wellentäler aufweist. Die Höhe der Wellenberge beziehungsweise Wellentäler bezogen auf die Öffnungsebene kann sich optional in Abhängigkeit eines Öffnungsdurchmessers der Öffnung je nach eingeführtem Schaft ändern. Im Optimalfall geht bei einer maximal weit aufgeweiteten Öffnung die ursprüngliche Wellenlinie in eine kreisringförmige Linie über.

Um einen optimalen Schutz des Dichtungselements zu erreichen, ist es vorteilhaft, wenn die freien Enden der Rückhalteelemente tragenden Schutzelemente zwischen die Wellenberge und Wellentäler eingreifen. Die Schutzelemente schützen so das Dichtelement bis an die Wellenlinie heran gegen eine Beschädigung.

Die Herstellung des Abdichtungssystems wird besonders einfach, wenn die Schutzvorrichtung mit dem Dichtelement lösbar verbindbar ist. So kann zudem sichergestellt werden, dass die Schutzvorrichtung in optimaler Weise am Dichtelement positioniert werden kann, um dieses optimal vor eingeführten Instrumenten zu schützen. Außerdem kann so das Dichtelement einfach ausgetauscht werden.

Eine einfache Verbindung der Schutzvorrichtung mit dem Dichtelement kann insbesondere dadurch erreicht werden, dass das Dichtelement zu den Verbindungselementen der Verbindungsvorrichtung korrespondierende Verbindungsglieder aufweist. Die Verbindungsglieder können in Form von Vorsprüngen und/oder Ausnehmungen ausgebildet sein, in welche die Verbindungselemente vorzugsweise formschlüssig eingreifen.

Um eine optimale Abdichtung von Instrumentenschäften zu erreichen, auch wenn diese bezogen auf eine Längsachse des Abdichtungssystems schräg eingeführt werden, ist es vorteilhaft, wenn die Verbindungsglieder am Dichtelement bezogen auf eine quer zur Längsachse desselben verlaufende Ebene verkippbar, verschwenkbar oder neigbar angeordnet sind. Diese Ausgestaltung hat den Vorteil, dass so auch eine am Dichtelement gelagerte Schutzvorrichtung ihre Funktion insbesondere beim Auslenken eines Instruments optimal ausüben kann. Die in den Verbindungsgliedern gehaltenen Verbindungselemente führen dazu, dass auch der Grundkörper der Schutzvorrichtung entsprechend verschwenkt, verkippt oder geneigt wird, wenn das Dichtelement verkippt wird. Umgekehrt kann auch die Schutzvorrichtung bei einem Verkippen von Instrumenten das Dichtelement entsprechend mitneigen beziehungsweise eine Auslenkbewegung des gesamten Dichtelements unterstützen. Insbesondere bei einer Auffaltung eines oben beschriebenen Dichtelements mit wellenförmig gefalteter Wand ist dies vorteilhaft, um aufgrund geringer radialer Umfangspannungen das Dichtelement nicht aufzuziehen.

Vorteilhafterweise sind die Verbindungselemente und Verbindungsglieder in Umfangsrichtung korrespondierend zur Wellenlinie angeordnet. Auf diese Weise kann sichergestellt werden, dass freie Enden der Schutzelemente der Schutzvorrichtung nur bis zur Wellenlinie heranreichen und diese nicht überdecken, was eine Abdichtung eines eingeführten Instrumentenschafts unmöglich machen würde. Zudem kann so auch sichergestellt werden, dass die Schutzelemente stets bis an die Wellenlinie heranreichen, beispielsweise durch Eintauchen ihrer freien Enden in Wellentäler, und so dass Dichtelement im Wesentlichen vollständig vor Beschädigungen schützen können.

Günstigerweise umfasst das Abdichtungssystem eine Dichtelementhalterung zum Halten des Dichtelements, welche Dichtelementhalterung mit der Trokarhülse lösbar verbindbar ist. Insbesondere ist es so möglich, das Dichtelement vor dem Einsetzen in die Trokarhülse oder in eine entsprechende Aufnahme einer Dichtelementhalterung zu halten, was eine Handhabung des Abdichtungssystems insgesamt verbessert. Ferner wird so auch eine Montage des Abdichtungssystems erleichtert.

Vorzugsweise weist die Trokarhülse eine Dichtelernenthalterungsaufnahme auf zum Einsetzen der Dichtelementhalterung. Es ist so möglich, die Teile des Abdichtungssystems einfach und sicher zusammenzusetzen.

Günstigerweise umfasst die Dichtelementhalterung ein Halterungsdichtelement zum Abdichten der Dichtelementhalterung mit Bezug zu einer inneren Wandfläche der Trokarhülse. Auf diese Weise können Fluidverluste, insbesondere Gasverluste, durch das Abdichtungssystem hindurch verhindert werden. Ein durch die Trokarhülse definierter Fluidkanal wird so optimal abgedichtet, zum einen durch das Halterungsdichtelement und zum anderen durch das in die Dichtelementhalterung eingesetzte Dichtelement. Die Dichtelementhalterung hat ferner den Vorteil, dass insbesondere Verschleißteile des Dichtungssystems, zum Beispiel das Dichtelement, auf einfache Weise ausgetauscht werden können.

Auf einfache und sichere Weise kann eine Abdichtung der Dichtelementhalterung relativ zur Trokarhülse erreicht werden, wenn das Halterungsdichtelement an einer in proximaler oder im Wesentlichen in proximaler Richtung weisenden Ringfläche der Trokarhülse anliegt.

Besonders einfach wird der Aufbau des Abdichtungssystems, wenn die Ringfläche durch eine einstufige Durchmesserverjüngung eines Innendurchmessers der Trokarhülse definiert ist. Insbesondere kann die Durchmesserverjüngung in Richtung auf ein distales Ende der Trokarhülse hin ausgebildet sein.

Besonders einfach in der Herstellung wird das Abdichtungssystem, wenn das Halterungsdichtelement einstückig mit der Dichtelementhalterung ausgebildet ist. Insbesondere kann das Halterungsdichtelement, wie die gesamte Dichtelementhalterung, aus einem Kunststoff hergestellt sein, so dass die Dichtelementhalterung insgesamt beispielsweise aus einem Kunststoff als ein Teil gespritzt werden kann. Dies vereinfacht zudem den Zusammenbau des Abdichtungssystems, da kein zusätzliches Bauteil erforderlich ist und montiert werden muss, um die Dichtelementhalterung relativ zur Trokarhülse abzudichten. Das Einsetzen der Dichtelementhalterung mit einem darin gehaltenen Dichtelement in die Trokarhülse reicht zur Abdichtung aus. Keine weiteren Schritte sind hierfür nötig.

Vorzugweise ist das Halterungsdichtelement in Form eines in radialer Richtung von der Dichtelementhalterung abstehenden Flansches ausgebildet. Ein derartiger Flansch kann auf einfache Weise hergestellt werden und insbesondere durch entsprechendes Anlegen an eine quer oder im Wesentlichen quer zur Längsachse des Abdichtungssystems verlaufende, optional auch geneigte, Ringfläche eine optimale Abdichtung der Trokarhülse relativ zur Dichtelementhalterung sicherstellen.

Um insbesondere Fertigungstoleranzen bei der Herstellung der Teile des Abdichtungssystems ausgleichen zu können, ist es günstig, wenn der Flansch etwas in distaler Richtung weisend bezogen auf eine quer zur Längsachse verlaufende Ebene geneigt ist. Insbesondere kann so das Halterungsdichtelement mit Vorspannung gegen eine entsprechende (Dicht-)Fläche der Trokarhülse gedrückt werden beim Einsetzen der Dichtelementhalterung.

Eine besonders gute Abdichtung der Dichtelementhalterung relativ zur Trokarhülse, unabhängig von etwaigen Fertigungstoleranzen einzelner Teile des Abdichtungssystems, kann erreicht werden, wenn das Halterungsdichtelement mindestens abschnittsweise elastisch verformbar ist. Insbesondere kann ein Rand des Halterungsdichtelements eine Dichtlippe bilden und an einer beliebigen Innenfläche der Trokarhülse anliegen.

Günstigerweise ist das Dichtelement aus einem Kunststoff hergestellt, vorzugsweise aus einem dampf- und/oder gamma-sterilisierbaren Kunststoff. Vorteilhaft ist es, wenn der Kunststoff ein Elastomer ist oder enthält, insbesondere Silikon oder Polyisopren. Dadurch können die Rückhalteelemente der Schutzvorrichtung besonders gut mit dem Dichtelement in Eingriff gebracht werden.

Um eine Verletzung des Dichtelements zu vermeiden und trotzdem eine für das Vermeiden einer Relativbewegung zwischen dem Dichtelement und der Schutzvorrichtung erforderliche Verformung des Dichtelements durch die Rückhalteelemente zu gestatten, ist es vorteilhaft, wenn das Dichtelement aus einem Material mit einer Härte in einem Bereich von etwa 20 Shore A bis etwa 60 Shore A aufweist.

Vorzugsweise weist das Dichtelement eine Wandstärke in einem Bereich von etwa 0,2 mm bis etwa 0,8 mm auf. Ein Dichtelement mit einer Wandstärke im angegebenen Bereich gestattet es, in der oben beschriebenen gewünschten Weise von den Rückhalteelementen verformt oder ausgebeult zu werden, um eine Relativbewegung zwischen dem Dichtelement und der Schutzvorrichtung im Bereich der Rückhalteelemente zu vermeiden.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Gesamtansicht eines chirurgischen Abdichtungssystems;
- Figur 2:: eine Schnittansicht längs Linie 2-2 in Figur 1;
- Figur 3:: eine vergrößerte Teilansicht der Schnittansicht in Figur 2;
- Figur 4:: eine perspektivische Explosionsdarstellung des Abdichtungssystems aus Figur 1;
- Figur 5:: eine perspektivische Ansicht des Dichtungselements aus Figur 4;
- Figur 6:: eine Schnittansicht längs Linie 6-6 in Figur 3;
- Figur 7:: eine perspektivische Explosionsdarstellung eines Dichtelements mit Schutzvorrichtung;
- Figur 8:: eine Schnittansicht längs Linie 8-8 in Figur 7;
- Figur 9:: eine Schnittansicht längs Linie 9-9 in Figur 7;
- Figur 10:: eine perspektivische Ansicht der Schutzvorrichtung in einer maximal aufgeweiteten Stellung;
- Figur 11:: eine Schnittansicht analog Figur 3 beim Einführen eines Obturators des Abdichtungssystems;
- Figur 12:: eine Ansicht analog Figur 1 des Abdichtungssystems mit eingesetztem Obturator;
- Figur 13:: eine Längsschnittansicht des in Figur 12 dargestellten Obturators; und

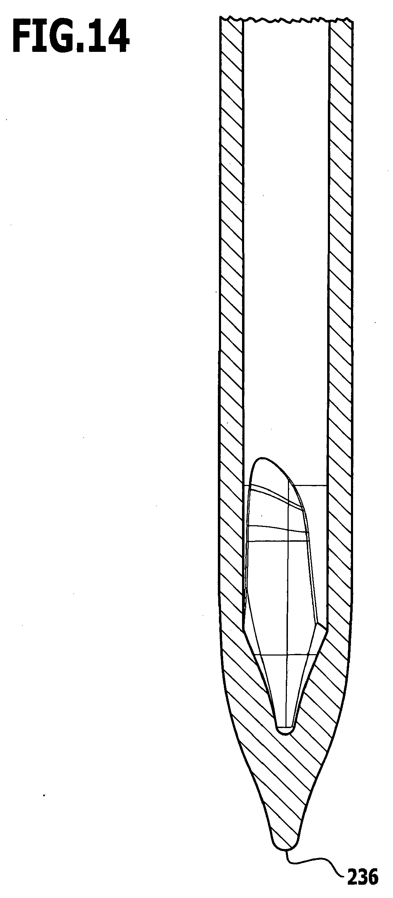

- Figur 14:: eine Schnittansicht längs Linie 14-14 in Figur 13;
- Figur 15:: eine schematische Darstellung der Funktionsweise der Schutzvorrichtung und Rückhalteelemente derselben in einer Schnittansicht ähnlich dem Ausschnitt C in Figur 6; und
- Figur 16:: eine schematische Darstellung analog Figur 15 bei Fehlfunktion der Rückhalteelemente der Schutzvorrichtung.

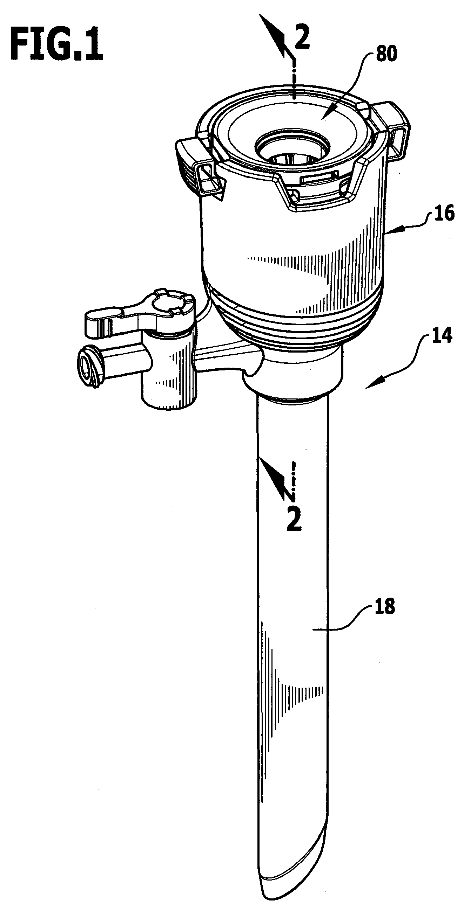

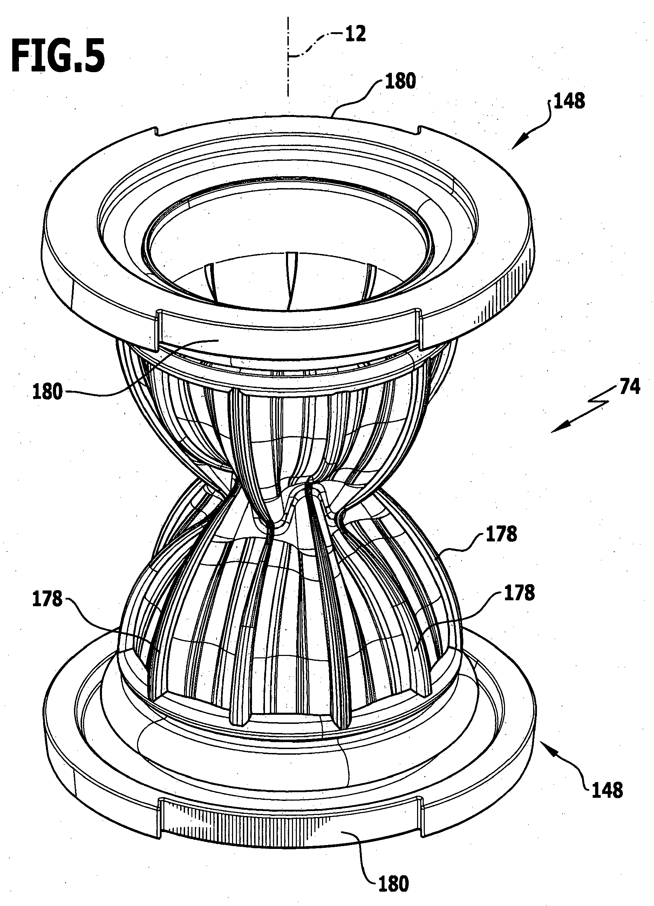

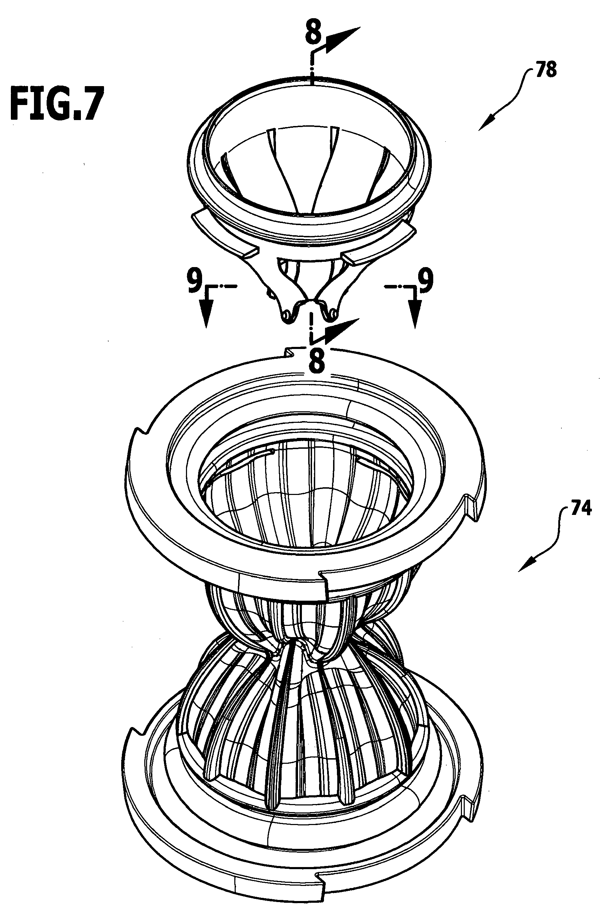

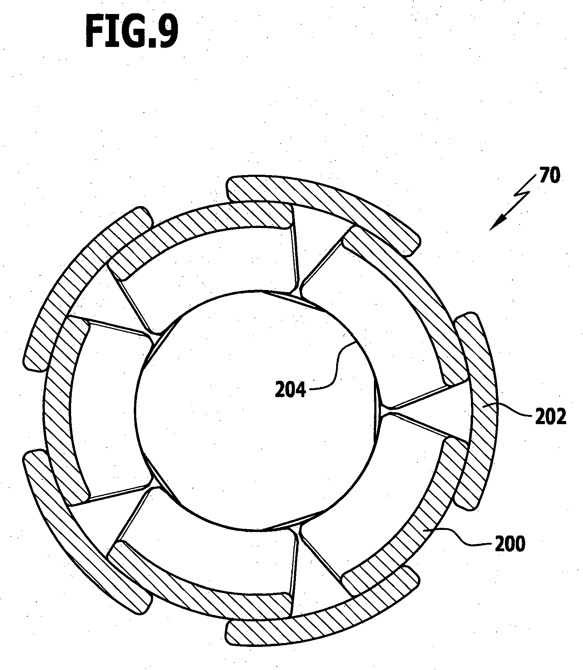

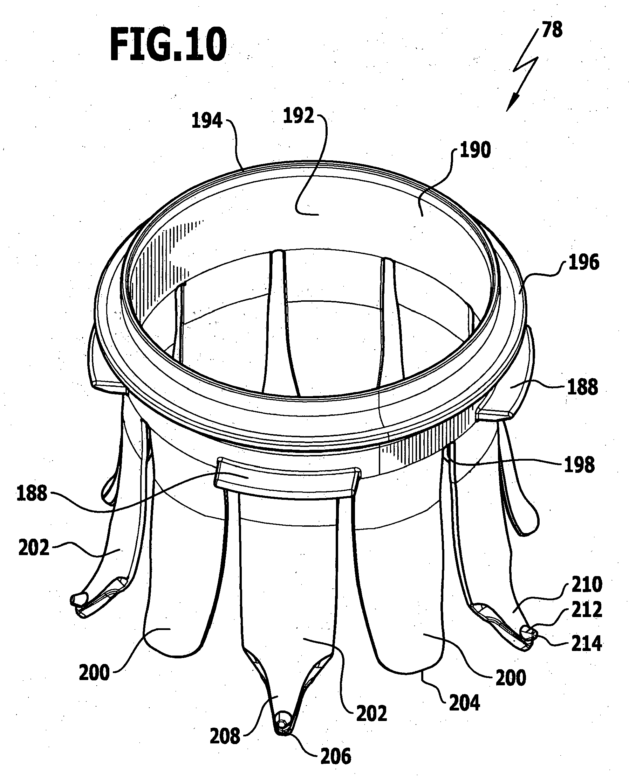

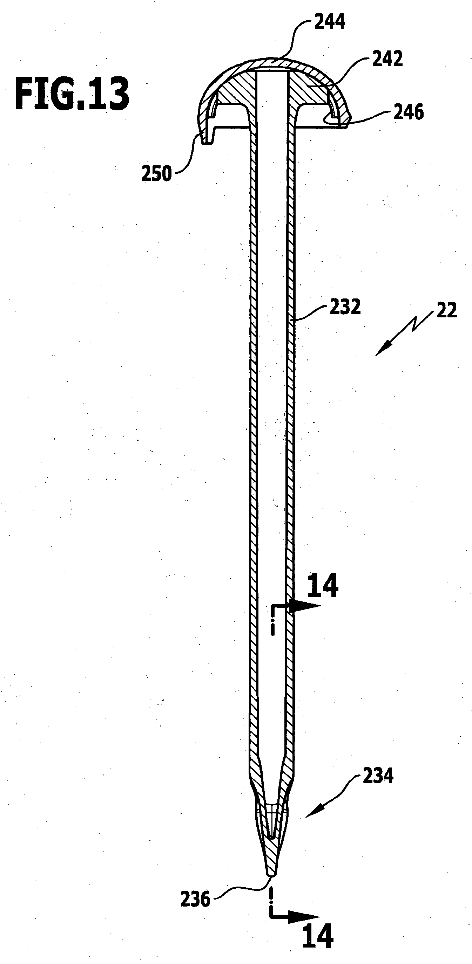

In den Figuren 1 bis 13 ist ein insgesamt mit dem Bezugszeichen 10 versehenes, ein chirurgisches Abdichtungssystem bildendes Trokarsystem dargestellt. Es umfasst eine eine Längsachse 12 definierende Trokarhülse 14 mit einem Dichtungsgehäuse 16 und einem sich von diesem weg in distaler Richtung erstreckenden Schaft 18, eine im Dichtungsgehäuse 16 angeordnete Dichtungsanordnung 20 sowie einen Obturator 22, welcher ein speziell zum Trennen und Aufweiten von Körpergewebe geformtes distales Ende aufweist und vor dem Einführen der Trokarhülse 14 in einen Patientenkörper in die Trokarhülse 14 eingeschobenen wird, um das Einführen der Trokarhülse 14 in den Patientenkörper zu Erleichtern.

Die Trokarhülse 14 ist im Wesentlichen rotationssymmetrisch ausgebildet und definiert im Inneren des Dichtungsgehäuses 16 eine Aufnahme 24 für die Dichtungsanordnung 20. Ein minimaler Innendurchmesser der Trokarhülse 14 wird definiert durch den Schaft 18. In einem ersten Übergangsbereich 26 vom Schaft 18 zum Dichtungsgehäuse 16 erweitert sich der Innendurchmesser des Schafts 18 kontinuierlich und bleibt im Bereich eines ersten Erweiterungraums 28 konstant. An den ersten Erweiterungsraum 28 schließt sich ein zweiter Übergangsbereich 30 an, in dem sich der Inneridurchmesser der Trokarhülse 14 nochmals kontinuierlich erweitert bis zu einem distalen Teil 32 der Aufnahme 24.

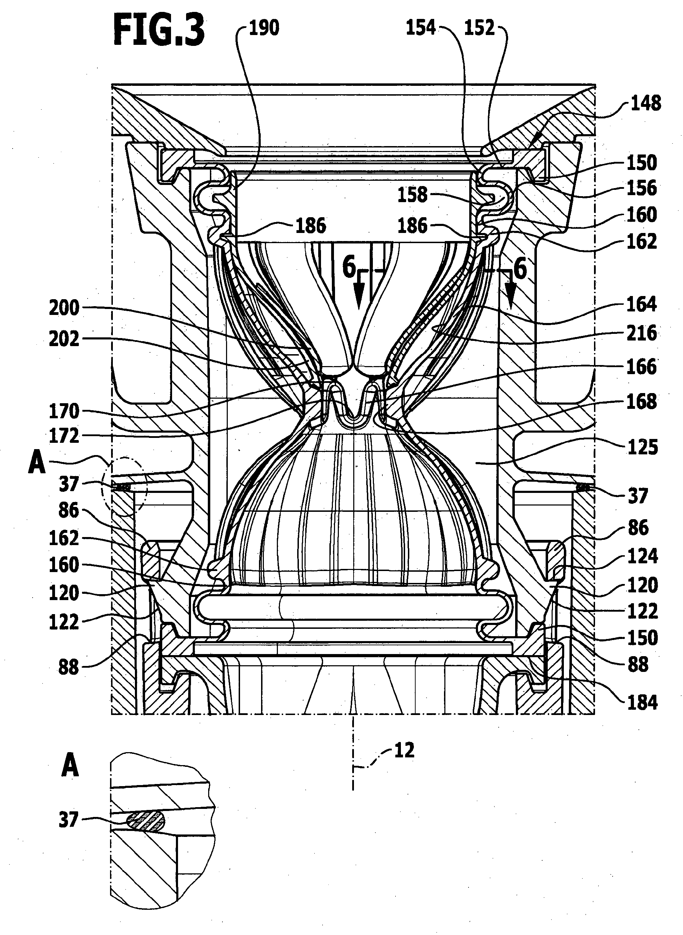

Ein Innendurchmesser des Dichtungsgehäuses 16 erweitert sich einstufig beim Übergang vom distalen Teil 32 zu einem proximalen Teil 34 desselben, so dass eine in proximaler Richtung weisende Ringfläche 36 definiert wird. Die Ringfläche kann optional eine zusätzliche Dichtung 37 tragen, welche durch Anspritzen eines Elastomers hergestellt und beispielhaft in Figur 3 punktiert eingezeichnet ist. Eine flache Ausnehmung 38 in der Ringfläche definiert somit eine etwas in proximaler Richtung vorstehende ebene Dichtfläche 40, welche von einer Innenwand 42 des proximalen Teils 34 durch die Ausnehmung 38 getrennt ist.

Ausgehend von einem proximalen Ende 44 des Dichtungsgehäuses 16 sind diametral bezogen auf die Längsachse 12 einander gegenüberliegend zwei zueinander symmetrische Verriegelungsaufnahmen 46 ausgebildet, welche jeweils zwei seitliche Hinterschneidungen 48 aufweisen, welche in Umfangsrichtung in einander entgegengesetzte Richtungen geöffnet sind. Die Verriegelungsaufnahmen 46 bilden einen Teil einer Rastverbindung, mit welcher die Dichtungsanordnung 20 im Dichtungsgehäuse 16 verriegelt werden kann, wie nachfolgend noch im Einzelnen noch erläutert wird.

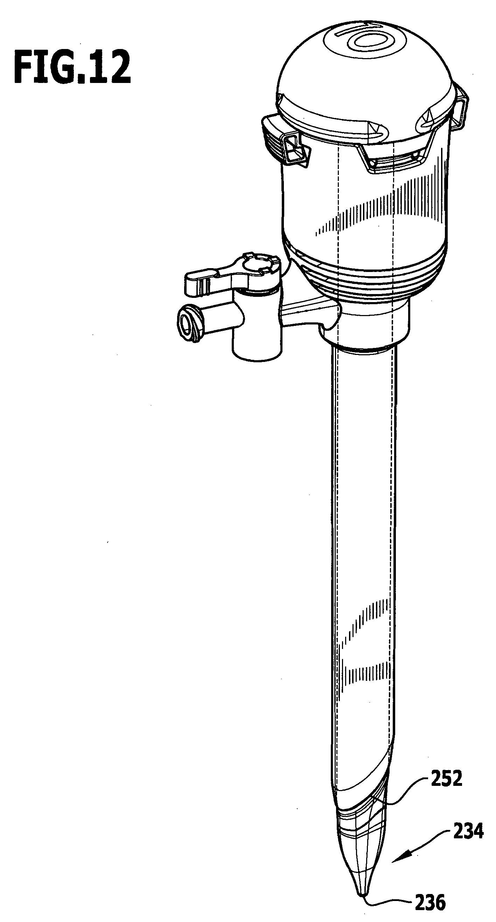

Ferner ist ausgehend vom Ende 44 in einer Wand 52 des Dichtungsgehäuses 16 eine sich in distaler Richtung etwas verjüngende Aussparung 50 symmetrisch zwischen den Verriegelungsaufnahmen 46 ausgebildet, in welche ein entsprechender Vorsprung 250 des Obturators 22 eingreift, wenn der Obturator 22 vollständig in die Trokarhülse 14 eingeführt ist, wie dies in Figur 12 dargestellt ist.

Am Dichtungsgehäuse 16 ist im Bereich des zweiten Übergangsbereichs 30 einseitig ein Anschlussstutzen 54 angeformt, welcher einen senkrecht zur Längsachse 12 verlaufenden Kanal 56 definiert. In den Kanal 56 eingesteckt ist ein Stutzen 57 eines Verschlusselements 58 mit einem in entgegengesetzter Richtung abstehenden, standardisierten Luer-Lock-Anschluss 60. Das Verschlusselement 58 umfasst ein zylindrisches Ventilgehäuse 62, in welchen ein korrespondierend ausgebildeter zylindrischer Verschlusskolben 64 mit einem angeformten Betätigungshebel 66 eingesetzt ist. Der Verschlusskolben 64 ist mit einer Bohrung 68 versehen, so dass in Abhängigkeit einer Verdrehstellung des Verschlusskolbens 64 relativ zum Ventilgehäuse 62 den Kanal 56 für Fluide geöffnet oder geschlossen werden kann. Statt des beschriebenen Verschlusselements 58 können auch beliebige andere Arten bekannter Verschlusselemente vorgesehen sein, insbesondere auch spezielle, federbelastete und dadurch selbstschließende Luer-Lock-Anschlüsse.

Die Dichtungsanordnung 20 umfasst zwei Dichtungen, nämlich ein Kreuzschlitzventil 70, welches an einem Haltering 72 gehalten ist, sowie ein Dichtelement 74, welches in seinem prinzipiellen Aufbau im deutschen Gebrauchsmuster 20 2006 005 442 detailliert beschrieben ist.

Das Dichtelement 74 ist im Inneren einer Dichtelementhalterung 76 gehalten, welche mit dem Haltering 72 lösbar verbindbar ist. Proximalseitig ist am Dichtelement 74 eine Schutzvorrichtung 78 lösbar gehalten, so dass das Dichtelement 74 zusammen mit der Schutzvorrichtung 78 bei Bedarf aus der Dichtelementhalterung 76 entnommen werden kann. Proximalseitig kann die Dichtelementhalterung 76 noch mit einem Deckel 80 verschlossen werden.

Die einzelnen Teile der Dichtungsanordnung 20 werden nachfolgend im Einzelnen näher beschrieben.

Der Haltering 72 umfasst einen im Querschnitt kreisförmigen Ring 82, von dessen proximalseitigem Rand ein in proximaler Richtung abstehender ringförmiger Flansch 84 ausgebildet ist, welcher sich jedoch nicht über eine gesamte Breite einer Wand des Rings 82 erstreckt, sondern nur etwa über die Hälfte. Vom proximalen Rand des Rings 82 stehen ferner zwei zueinander symmetrisch ausgebildete Verbindungsflügel 86 einander, bezogen auf die Längsachse 12, diametral gegenüberliegend in proximaler Richtung ab. Die Verbindungsflügel 86 weisen jeweils zwei im Wesentlichen rechteckige Durchbrechungen 88 auf, welche quer zur Längsachse 12 orientiert sind. Die Verbindungsflügel 86 sind vom Flansch 84 etwas beabstandet angeordnet, so dass zwischen dem Flansch 84 und den Verbindungsflügeln 86 jeweils eine Nut 90 ausgebildet ist.

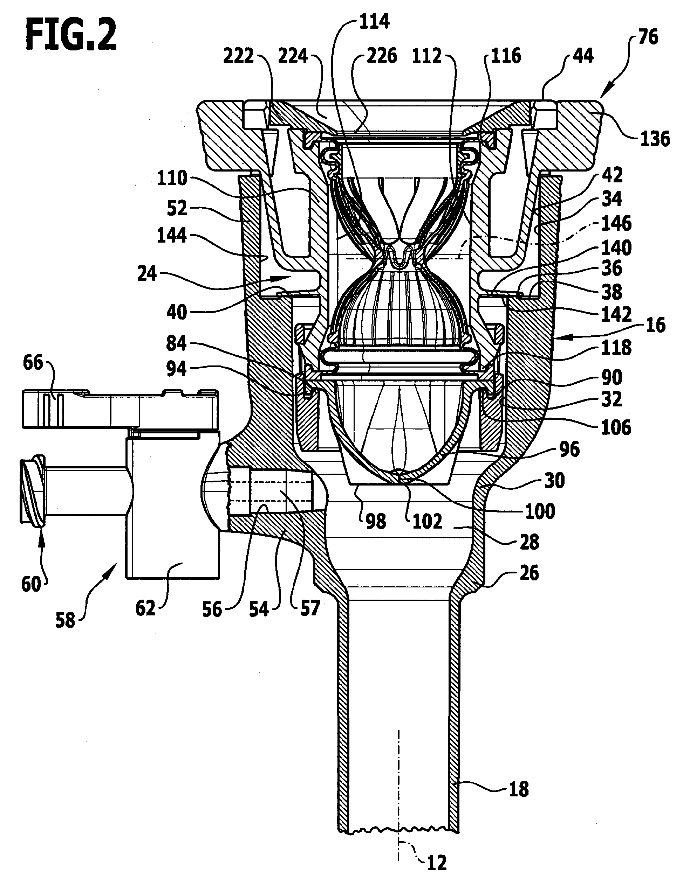

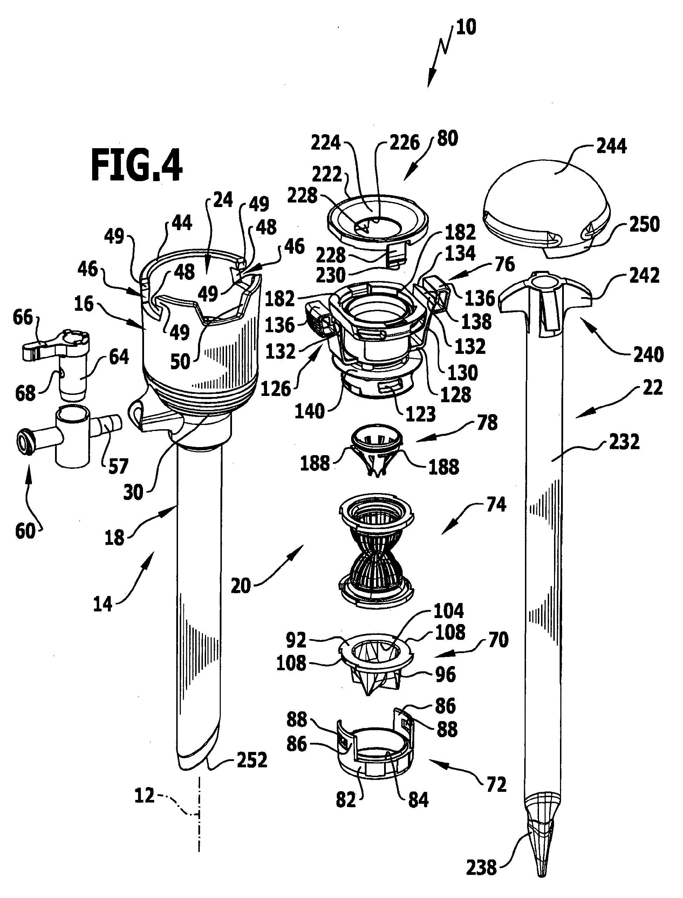

Das Kreuzschlitzventil 70 umfasst proximalseitig einen ringförmigen Befestigungsflansch 92, welcher einen in distaler Richtung weisenden Ringvorsprung 94 trägt, welcher in seiner Höhe sowie in seinen äußeren Abmessungen korrespondierend zu den Nuten 90 ausgebildet ist. Das Kreuzschlitzventil 70 umfasst ferner einen am Befestigungsflansch 92 in distaler Richtung abstehenden Ventilkörper 96, welcher distalseitig in eine kreuzförmige Endfläche 98 mündet, die mit zwei zueinander senkrechten Schlitzen 100 versehen ist. Der Ventilkörper 96 ist in einer Grundstellung, wie sie beispielsweise in den Figuren 2 und 4 dargestellt ist, so ausgebildet, dass die durch die Schlitze 100 getrennten Schnittflächen 102 des Ventilkörpers 96 direkt aneinander anliegen und so eine durch den Befestigungsflansch 92 definierte ringförmige Öffnung 104 etwas distalseitig des Befestigungsflanschs 92 vollständig verschließen. Der Ventilkörper 96 ist proximalseitig direkt an einen Innenrand des Befestigungsflansch 92 angeordnet ist, so dass zwischen dem Ventilkörper 96 und dem Ringvorsprung 94 eine Ringnut 106 ausgebildet wird, in welche der Flansch 84 im Wesentlichen formschlüssig eingreifen kann.

Der Befestigungsflansch 92 ist ferner mit zwei in radialer Richtung weisenden Aussparungen 108 versehen, in welche die Verbindungsflügel 86 eingreifen, wenn der Ventilkörper 96 in den ,Haltering 72 eingesetzt ist und mindestens teilweise mit dem Ventilkörper 96, insbesondere mit dessen die Schlitze 100 aufweisender Endfläche 98, über einen distalseitigen Rand des Rings 82 vorsteht.

Die Dichtelementhalterung 76 ist im Wesentlichen langgestreckt hülsenförmig ausgebildet. Sie umfasst einen zentralen, koaxial zur Längsachse 22 ausgebildeten Hülsenkörper 110. Eine Innenfläche 112 des Hülsenkörpers 110 ist vollständig rotationssymmetrisch ausgebildet. Die Innenfläche 112 definiert und begrenzt einen Längskanal 114, in welchen das Dichtelement 74 eingesetzt ist. Ein Innendurchmesser des Hülsenkörpers 110 erweitert sich jeweils zum distalen und proximalen Ende desselben hin etwas. Distalseitig und proximalseitig sind jeweils ringförmige, in proximaler beziehungsweise distaler Richtung weisende Ringvorsprünge 116 und 118 ausgebildet, zum in Eingriff Bringen mit korrespondierenden Flanschen beziehungsweise Nuten am Dichtelement 74.

Auf einer Außenseite des Hülsenkörpers 110 sind etwas proximalseitig des distalseitigen Ringvorsprungs 118 zwei bezogen auf die Längsachse 12 einander diametral gegenüberliegende und in entgegengesetzte Richtungen weisende Rastnasen 120 ausgebildet, welche nach außen weisende, etwas in distaler Richtung geneigte Aufgleitflächen 122 und somit auch eine in proximaler Richtung weisende Ringkante 124 definieren. Die Rastnasen 120 sind korrespondierend zu den Durchbrechungen 88 an den Verbindungsflügeln 86 ausgebildet. Die Verbindungsflügel 86 können von distal her kommend über die Aufgleitflächen 122 geschoben werden, so dass sie etwas in radialer Richtung von der Längsachse 12 weg nach außen ausschwenken. Sobald die Rastnasen 120 ganz in die Durchbrechungen 88 eingreifen können, federn die Verbindungsflügel 86 wieder in Richtung auf die Längsachse 12 zurück. Auf die beschriebene Weise können der Haltering 72 und die Dichtelementhalterung 76 rastend miteinander verbunden werden.

Zwischen den Rastnasen 120, also um 90° relativ zu diesen in Umfangsrichtung versetzt, sind im Hülsenkörper 110 zwei rechteckige Durchbrechungen 123 vorgesehen, welche einen Innenraum 125 des Hülsenkörpers 110 mit einer Außenseite desselben verbinden. Auf diese Weise kann ein Druckausgleich zwischen dem Innenraum 125 und einer Umgebung der Dichtelementhalterung 76 erreicht werden. Der so erreichbare Druckausgleich zwischen einem im Körper eines Patienten herrschenden Gasdrucks und dem Innenraum 125 bzw. die so mögliche Ent-/Belüftung des Innenraums 125 verhindert, dass eine Aufweitung des Dichtelements 74 gegen ein Gasvolumen im Innenraum erfolgen muss, welches nach Montage des Dichtelements 74 an der Dichtelementhalterung 76 eingeschlossen würde.

Zum Verbinden der Dichtelementhalterung 76 mit dem Dichtungsgehäuse 16 sind von einer Außenseite der Dichtelementhalterung 76 zwei Kupplungsglieder 126 einander diametral gegenüberliegend abstehend angeordnet. Sie umfassen jeweils einen direkt vom Hülsenkörper 110 in radialer Richtung abstehenden Quersteg 128, von welchem sich ein im Wesentlichen parallel zum Hülsenkörper 110 in proximaler Richtung erstreckender Federteil 130 weg erstreckt. An einem proximalen Ende des Federteils 130 sind beidseits des Federteils 130 im Wesentlichen in Umfangsrichtung weisende und überstehende Rastvorsprünge 132 ausgebildet, welche jeweils von der Längsachse 12 weg weisende Aufgleitflächen 134 definieren. Zwischen den Aufgleitflächen 134 ist auf einer Außenseite der Federteile 130 ein im Wesentlichen quaderförmiges Bedienelement 136 angeordnet, welches proximalseitig etwas über das Ende des Federteils 130 vorsteht.

Zum Verbinden der Dichtelementhalterung 76 mit dem Dichtungsgehäuse 16 wird das distale Ende der Dichtelementhalterung 76 in das Dichtungsgehäuse 16 eingeführt, bis die die Hinterschneidungen 46 seitlich begrenzende Vorsprünge 49 mit den Aufgleitflächen 134 in Kontakt kommen und die Federteile 130 infolge des Aufgleitens etwas in Richtung auf die Längsachse 12 hin verschwenken. Sobald eine proximale Endfläche 138 der Federteile 130 in die Hinterschneidung 48 eintauchen kann, federn die Federteile 130 in radialer Richtung etwas nach außen und die Endfläche 130 liegt an einer in distaler Richtung weisenden Kante des Vorsprungs 49 an. Zum Lösen der Dichtelementhalterung 76 von der Trokarhülse 14 können die Bedienelemente 136 mit einer in Richtung auf die Längsachse 12 wirkenden Kraft beaufschlagt werden, so dass die Federteile 130 in Richtung auf die Längsachse 12 verschwenkt werden und die Rastvorsprünge 132 wieder die Hinterschneidung 48 freigeben. Die Dichtelementhalterung 76 kann dann in proximaler Richtung aus dem Dichtungsgehäuse 16 herausgezogen werden.

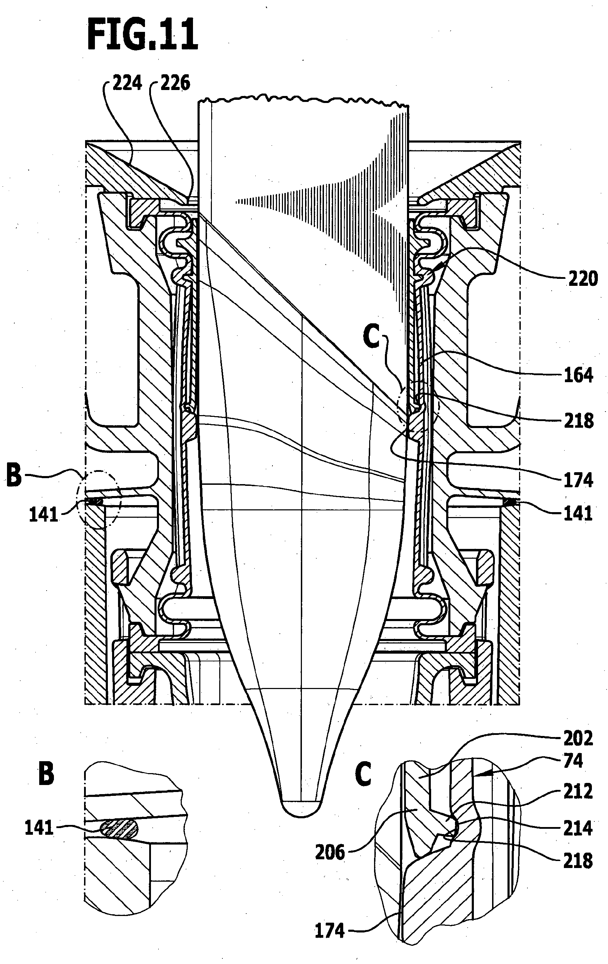

Etwas distalseitig der Querstege 128 ist ein Halterungsdichtelement 140 ausgebildet, und zwar in Form eines im Wesentlichen in radialer Richtung abstehenden Ringflansches, welcher etwas in distaler Richtung geneigt ist, und zwar um etwa 2° bezogen auf eine senkrecht zur Längsachse 12 verlaufende Querebene. Das Halterungsdichtelement 140 weist eine Dicke auf, welche eine gewisse Elastizität beziehungsweise Flexibilität des Halterungsdichtelements 140 vorgibt. Es kann so in axialer Richtung etwas federn und Fertigungstoleranzen an der Trokarhülse 14 und der Dichtelementhalterung 76 ausgleichen. Das Halterungsdichtelement 140 ist an der Dichtelementhalterung 76 derart angeordnet, dass dann, wenn die Dichtelementhalterung 76 rastend in der beschriebenen Weise mit dem Dichtungsgehäuse 16 verbunden ist, eine in distaler Richtung weisende Dichtfläche 142 der Dichtelementhalterung 76 an der Ringfläche 36, optional etwas vorgespannt, anliegt und so eine perfekte Abdichtung der Dichtelementhalterung 76 bezogen auf eine Innenwand 144 des Dichtungsgehäuses 16 der Trokarhülse erreicht wird. Das Halterungsdichtelement 140 kann zur Verbesserung einer Abdichtungswirkung optional eine weitere Dichtung 141 tragen, welche durch Anspritzen eines Elastomers hergestellt und beispielhaft in Figur 11 punktiert eingezeichnet ist.

Das Dichtelement 74 ist bezogen auf die Längsachse 12 im Wesentlichen rotationssymmetrisch ausgebildet. Ferner ist es bezogen auf eine quer zur Längsachse verlaufende Öffnungsebene 146 im Wesentlichen spiegelsymmetrisch ausgebildet. Die Öffnungsebene 146 verläuft parallel zu zwei beidseitigen Flanschringen 148, welche das Dichtelement 74 distal- und proximalseitig begrenzen und einen maximalen Außendurchmesser des Dichtelements 74 definieren. Von den Flanschringen 148 stehen maximal weit außen an ihnen und jeweils in die Richtung des anderen Flanschrings 148 hin weisend Ringvorsprünge 150 ab, welche die Ringvorsprünge 116 und 118 außen umgreifen können. Das Dichtelement 74 kann so auf einfache Weise über die Ringvorsprünge 116 und 118 gehängt oder gespannt und im Inneren der Dichtelementhalterung 76 gehalten werden.

Von den Flanschringen 148 erstreckt sich in radialer Richtung auf die Längsachse 12 hin ein erster Querabschnitt 152, welche in einen ersten, nach außen zurückgebogenen ersten Wulstabschnitt 154 übergeht, welcher wiederum direkt in einen zweiten Wulstabschnitt 156 übergeht, welcher wiederum ein auf die Längsachse 12 hin gerichtetes Ende aufweist. Der zweite Wulstabschnitt 156 definiert so eine in Richtung auf die Längsachse hin geöffnete Ringnut 158.

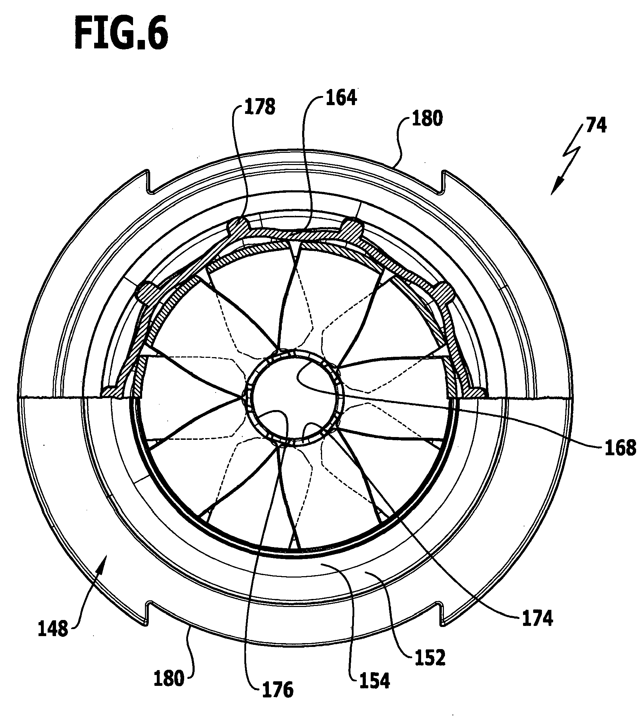

Distalseitig schließt sich an den zweiten Wulstabschnitt 156 ein kurzer zylindrischer Abschnitt 160 an, welcher in einen verdickten, außen am Dichtelement 74 abstehenden Wulst 162 übergeht. Ausgehend von den Wulsten 162, an denen eine Wand 164 des Dichtelements 74 im Wesentlichen faltenfrei absteht, faltet sich die Wand 164 gardinenartig bis zur Öffnungsebene 146 hin. Durch die Faltung entsteht eine Dichtlinie 166, welche in Form einer Wellenlinie Wellenberge 170 proximalseitig der Öffnungsebene 146 und Wellentäler 172 distalseitig der Öffnungsebene 146 definiert. Die Wellenlinie 168 ist etwas verstärkt und in Form einer Dichtlippe 174 ausgebildet, welche sich somit teilweise proximalseitig und teilweise distalseitig der Öffnungsebene 146 befindet. In einer Draufsicht, wie in Figur 6 dargestellt, ist jedoch erkennbar, dass die Dichtlinie 166 und damit auch die Dichtlippe 174 eine kreisringförmige Öffnung 176 des Dichtelements 74 begrenzen. Die Öffnung 176 weist in einer Grundstellung, wie sie beispielsweise in den Figuren 3 bis 6 dargestellt ist, einen minimalen Innendurchmesser auf. Die Öffnung 176 kann, wie beispielsweise in Figur 11 dargestellt, so weit aufgeweitet werden, dass ein Innendurchmesser derselben einem Innendurchmesser des Dichtelements 74 im Bereich der Wulste 162 entspricht. Die gefaltete Wand 164 entfaltet sich dabei, ist praktisch auf der gesamten Länge zwischen den Wulsten 162 vollständig umfaltet und definiert so eine im Wesentlichen zylindrische Wandfläche.

Zur Stabilisierung des Dichtelements 74 sind auf einer Außenseite der Wand 164 ausgehend von den Wulsten 164 bis an die Dichtlippe 166 heranreichende Verstärkungsrippen 178 ausgebildet. Das Dichtelement 74 ist insgesamt einstückig aus einem Kunststoff gespritzt, welcher vorzugsweise elastomerische Eigenschaften aufweist. Ferner sind an den Flanschringen 148 jeweils zwei, einander diametral gegenüberliegende Aussparungen 180 vorgesehen, welche korrespondierend zu zwei in radialer Richtung von der Dichtelementhalterung 76 in Richtung auf die Längsachse 12 hin vorstehenden Vorsprüngen 182 ausgebildet sind. Die Aussparungen 180 in Verbindung mit den Vorsprüngen 182 bilden eine Verdrehsicherung, so dass das Dichtelement 74 und die Dichtelementhalterung 76 in einer beispielsweise in den Figuren 2 und 3 dargestellten Montagestellung nicht um die Längsachse 12 relativ zueinander verdrehbar sind.

Nach Bestücken des Halterings 72 mit dem Kreuzschlitzventil 70 kann die Dichtelementhalterung 76, in welche das Dichtelement 74 in der oben beschriebenen Weise eingesetzt ist, mit dem Haltering 72 verbunden werden. Distalseitig bildet eine in distaler Richtung weisende ringförmige Stirnfläche 184 des Dichtelements 74 ein Anlagefläche für den Befestigungsflansch 92. Durch rastendes Verbinden des Halterings 72 mit der Dichtelementhalterung 76 in der oben beschriebenen Weise, werden der Befestigungsflansch 92 und der Flanschring 148 gegeneinander gedrückt und bilden eine perfekte Abdichtung.

Zumindest im proximalseitigen Wulst 162 sind vier gleichmäßig über den Umfang verteilte, in radialer Richtung auf die Längsachse 12 hin geöffnete und Verbindungsglieder bildende Vertiefungen 186 vorgesehen, welche der Aufnahme korrespondierender Verbindungselemente 188 der Schutzvorrichtung 78 dienen. Die Schutzvorrichtung 78 umfasst einen ringförmigen, in sich geschlossenen Grundkörper 190, welcher eine kreisförmige Durchbrechung 192 definiert. Vom Grundkörper 190 steht ein in radialer Richtung nach außen weisend benachbart einem proximalen Ende 194 ein Ringvorsprung 196 ab. Etwas weiter distalseitig sind die Verbindungselemente 188 in Form kurzer stegartiger Vorsprünge angeordnet. Sie erstrecken sich im Umfang über etwa 1/8 des Gesamtumfangs des Grundkörpers 190 und sind korrespondierend zu den Vertiefungen 186 ausgebildet. Der Grundkörper 190 kann somit direkt am Dichtelement 74 gelagert werden, wobei hierfür die Verbindungselemente 188 formschlüssig in die Vertiefungen 168 eingreifen. Sie bilden somit gleichzeitig eine Verdrehsicherung der Schutzvorrichtung 78 relativ zum Dichtelement 74. Ferner bilden sie auch eine Positionierhilfe der Schutzvorrichtung 78 relativ zum Dichtelement 74.

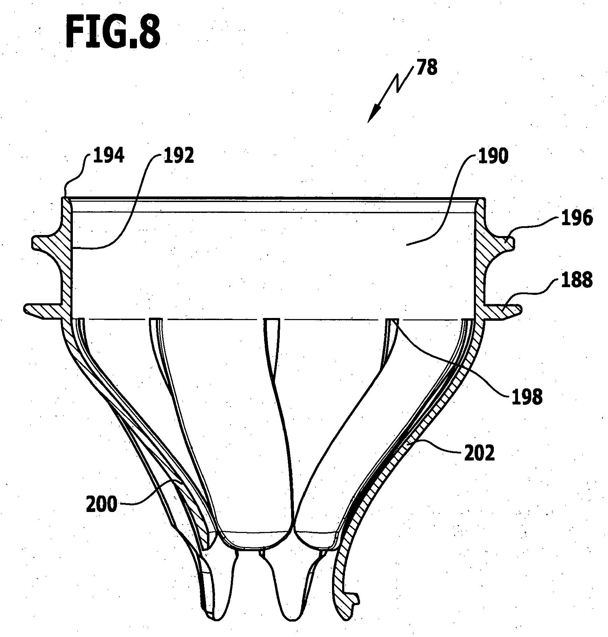

Von einem distalseitigen Rand 198 des Grundkörpers 190 erstrecken sich insgesamt 10, jeweils fünf lamellenförmige kurze Schutzelemente 200 und fünf lange Schutzelemente 202 in distaler Richtung. Sie weisen im Längsschnitt, wie in Figur 8 dargestellt, eine über ihre gesamte Länge konstante Dicke auf. Die kurzen Schutzelemente 200 sind im Wesentlichen bis zu ihrem freien Ende 204 nahezu gleich breit, die langen Schutzelemente 202 in etwa auf derselben Länge wie die kurzen Schutzelemente 200, allerdings nimmt dann eine Breite der langen Schutzelemente 202 auf deren distales Ende 206 hin deutlich ab, so dass ein schmaler Schutzelementabschnitt 208 ausgebildet wird, welcher in seiner Außenkontur im Wesentlichen korrespondierend zu einem Wellental 172 ausgebildet ist.

Die langen Schutzelemente 202 tragen jeweils ein von einer Außenseite 210 etwas in distaler Richtung geneigt abstehendes Rückhalteelement 212, welches eine Länge von weniger als 1mm aufweist. Das Rückhalteelement ist im Wesentlichen kegelstumpfförmig ausgebildet und weist eine abgerundete Spitze 214 auf.

Wenn der Grundkörper 190 in der oben beschriebenen Weise mit dem Dichtelement 174 verbunden ist, falten sich die aufgrund ihrer geringen Dicke flexiblen Schutzelemente 200 und 202 in Richtung auf die Längsachse 12 hin und nehmen die in den Figuren 6 bis 8 dargestellte Stellung ein. Es sei angemerkt, dass sich die langen Schutzelemente 202 distalseitig der Verbindungselemente 188 vom Rand 198 weg erstrecken, die kurzen Schutzelemente 200 in den Bereichen des Rands 198, zu welchem kein Verbindungselement 188 korrespondiert. Durch die entsprechend vorgesehenen Vertiefungen 186 kann die Schutzvorrichtung 78 positionsrichtig mit dem Dichtelement 74 verbunden werden, dies bedeutet, dass in einer Grundstellung alle fünf Schutzelementabschnitte 208 in korrespondierende Wellentäler 172 eintauchen. Damit ist sichergestellt, dass die distalen Enden 204 und 206 der Schutzvorrichtung 78 praktisch bis an die Dichtlinie 166 heranreichen und im Wesentlichen eine innere Wandfläche 216 des Dichtelements 74 vollständig verdecken.

Die Schutzelemente 200 und 202 stehen in der montierten Grundstellung bereits wenig distalseitig des Wulsts 162 von der Wandfläche 216 ab und berühren diese allenfalls nahe ihrer Enden 204 und 206. In der Grundstellung sind die Schutzelemente 200 und 202 einander überlappend angeordnet, wobei die kurzen Schutzelemente 200 näher an der Längsachse 12 liegen als die langen Schutzelemente 202. Dadurch berühren lediglich die Rückhalteelemente 212 benachbart der Dichtlinie 166 die Wand 164 des Dichtelements 74.

Wird ein Instrument, oder wie beispielsweise in Figur 11 dargestellt, der Obturator 22, von proximal her kommend in das Dichtelement 74 eingeführt, so tritt es zunächst in Kontakt mit Innenflächen der kurzen Schutzelemente 200. Ist ein Außendurchmesser, wie dies beim Obturator 22 der Fall ist, eines Instruments größer als die Öffnung 176, dann werden die kurzen Schutzelemente 200 gegen die langen Schutzelemente 202 gedrückt und nach außen verschwenkt. Dabei werden auch die Rückhalteelemente 212 mit ihren Spitzen 214 in die Wand 164 des Dichtelements 74 eingedrückt. Dies führt zu einer Auswölbung 218 der Wand 164 durch die Rückhalteelemente 212, so dass sich diese in der Wand 164 verhaken, man kann auch sagen, die Rückhalteelemente 212 und das Dichtelement 74 stehen miteinander in Eingriff. Durch das Verhaken der Rückhalteelemente 212 in der Wand 164 wird eine Relativbewegung der distalen Enden 206 der langen Schutzelemente 202 relativ zum Dichtelement 74 praktisch verhindert. Unabhängig von einer Aufweitung oder Auffaltung der Wand 164 in Abhängigkeit eines Durchmessers des eingeführten Instruments reichen die distalen Enden 206 der langen Schutzelemente 202 stets bis an die Dichtlinie 166 heran und schützen das Dichtelement 74 vor den eingangs beschriebenen möglichen Beschädigungen infolge des in Kontakt Tretens der Wand 164 mit spitzen Kanten der eingeführten Instrumente.

Selbst dann, wenn eine Längsachse des eingeführten Instruments relativ zur Längsachse 12 etwas verkippt wird, bleibt der Verhakeeffekt der Rückhalteelemente 212 erhalten. Durch eine von den Vertiefungen 186 und den Verbindungselementen 188 ausgebildete Verbindungseinrichtung 220 wird eine Verkippung des zunächst an der Schutzvorrichtung anliegenden Instrumentenschafts direkt auf das Dichtelement 74 übertragen, und zwar im Bereich des Wulstes 162, so dass das Dichtelement 74 analog zu einer Verkippung der Schutzvorrichtung 78 mit verkippt wird. Die besondere Anordnung der Schutzvorrichtung 78 am Dichtelement 74 bildet somit quasi auch eine Neigungsanpassung bei der Einführung von Instrumenten. Hierfür dienlich ist zudem insbesondere der erste Wulstabschnitt 154, welcher sowohl eine Verkippbewegung als auch eine transversale Bewegung, zumindest soweit der erste Wulstabschnitt 154 von einer Innenwand der Dichtelementhalterung 76 beabstandet ist, gestattet.

Durch die Wölbung der Schutzelemente 200 und 202 schwach konvex von der Wand 164 weg, ist sichergestellt, dass ein eingeführtes Instrument zunächst mit distalen Endbereichen der Schutzelemente 200 und 202 in Kontakt tritt, bevor es die Dichtlippe 174 berühren kann.

Zum Verschließen des Dichtungsgehäuses 16 dient der Deckel 80. Er umfasst einen ringförmigen Rahmen 222, von dem sich im Innern und in distaler Richtung eine sich im Durchmesser konisch verjüngende Deckelfläche 224 bis zu einer Deckelöffnung 226 erstreckt, welche einen maximalen Innendurchmesser der Dichtungsanordnung 20 definiert. Instrumente mit Schaftdurchmessern, welche größer sind als ein Innendurchmesser der Deckeöffnung 226, können nicht in die Trokarhülse 14 eingeführt werden. Der Deckel 80 weist ferner zwei in distaler Richtung weisende, einander gegenüberliegende Laschen 228 auf, welche an freien Enden Rastvorsprünge 230 aufweisen, welche mit korrespondierenden, in den Figuren nicht dargestellten Rastkanten an der Dichtelementhalterung 76 in Eingriff gebracht werden können. Der Deckel 80 kann dann nach Montage der Dichtelementhalterung 76 am Dichtungsgehäuse 16 auf einfache Weise auf die Dichtelementhalterung 76 aufgeschnappt werden.

Um die Trokarhülse 14 in einen menschlichen oder tierischen Körper einführen zu können, ist der Obturator 22 vorgesehen. Er umfasst einen hohlen, koaxial zur Längsachse 12 verlaufenden Schaft 232, welcher sich in einem distalen Endbereich 234 im Außendurchmesser kontinuierlich verjüngt und eine abgerundete Spitze 236 definiert. Der Endbereich 234 ist im Querschnitt an keiner Stelle kreisförmig, sondern in Folge definierter, sich parallel zur Längsachse 12 erstreckender Vertiefungen 238 unsymmetrisch ausgebildet. In einem proximalen Endbereich 240 sind an einer Außenseite des Schafts 232 vier jeweils 90° zueinander versetzt angeordnete Haltevorsprünge 242 vorgesehen, welche der Lagerung und Verbindung eines im Wesentlichen die Form einer Halbkugel aufweisenden Deckels 244 dienen. Am Deckel 244 sind auf einer Innenseite korrespondiere Vorsprünge 246 ausgebildet. Optional kann der Deckel 244 auch mit dem Schaft 232 und dessen Haltevorsprüngen 242 verschraubt oder verklebt sein. Des Weiteren steht vom Deckel 244 in distaler Richtung weisend der lappenförmige Vorsprung 250 ab, welcher korrespondierend zur Aussparung 50 ausgebildet ist, so dass der Obturator 22 mit einer definierten Orientierung bezogen auf die Längsachse 12 in die Trokarhülse 14 eingesetzt werden kann. Ist der Obturator 22 vollständig in die Trokarhülse 14 eingeschoben, steht der distale Endbereich 234 über eine bezogen auf die Längsachse 12 um etwa 45° geneigte Endfläche 252 des Schafts 18 vor, wie dies in Figur 12 dargestellt ist.

Im Inneren des Dichtungsgehäuses 16 erfolgt eine Abdichtung über das Dichtelement 74 sowie zu einem Außenbereich der Dichtelementhalterung 76 und der Innenwand 144 der Trokarhülse 14 mittels des Halterungsdichtelements 140. Wird der Obturator 22 aus der Trokarhülse 14 entfernt, verschließt das Kreuzschlitzventil 70 einen sich längs der Trokarhülse 14 erstreckenden Kanal fluiddicht. Aufgrund der etwas von der Längsachse 12 weg weisenden Außenflächen des Ventilkörpers 96 werden diese, falls im Körperinneren und somit im Bereich des Schafts 18 ein Überdruck besteht, die Schnittflächen 102 zusätzlich gegeneinander gedrückt, um die Schlitze 100 zu verschließen. Damit kann bei laparoskopischen Eingriffen, bei denen im Bauchraum eines Patienten mittels eines Gases ein Überdruck erzeugt wird, um einen Operationssitus freizuhalten, dieser Überdruck gehalten werden, auch dann, wenn Instrumente, oder zum Beispiel in analoger Weise der Obturator 22, mittels des Trokarsystems 10 ins Körperinnere eingeführt werden.

Zu beachten ist ferner, dass das Kreuzschlitzventil 70 erst mittels eines distalen Endes eines Instruments oder beispielsweise der Spitze 236 des Obturators 22 geöffnet werden kann, wenn beim Einführen eines Instruments dessen Schaft, beispielsweise der Schafts 232 des Obturators 22, mittels der Dichtlippe 144 des Dichtelements 74 abgedichtet ist. So ist sichergestellt, dass entweder das Kreuzschlitzventil 70 geschlossen oder eine Abdichtung mittels des Dichtelements 74 relativ zum eingeführten Instrument erfolgt.

Die Trokarhülse 14, der Haltering 72, das Kreuzschlitzventil-70, das Dichtungselement 74, die Schutzvorrichtung 78, die Dichtelementhalterung 76 sowie der Deckel 80 sind jeweils einstückig ausgebildet und vorzugsweise aus einem sterilisierbaren Kunststoffmaterial gespritzt. Der Obturator 22 ist wie beschrieben zweiteilig ausgebildet und kann ebenfalls an einem Kunststoffmaterial durch Spritzgießen hergestellt sein.

Über das Verschlusselement 58 kann bei entsprechender Stellung des Verschlusskolbens 64 ein Gas oder eine Flüssigkeit durch den Schaft 18 ins Innere eines Patientenkörpers eingeleitet oder auch abgeführt werden, selbst dann, wenn ein Instrument, beispielsweise der Obturator 22 in die Trokarhülse 14 eingeführt und ein durch die Trokarhülse 14 definierter Kanal proximalseitig des Anschlussstutzens 54 abgedichtet ist.

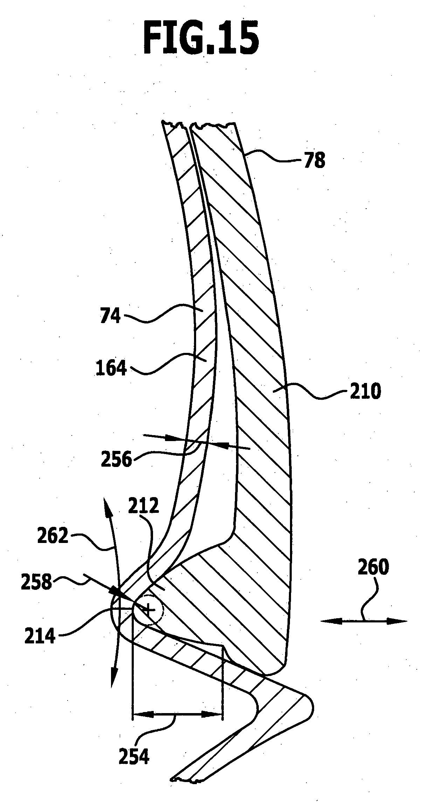

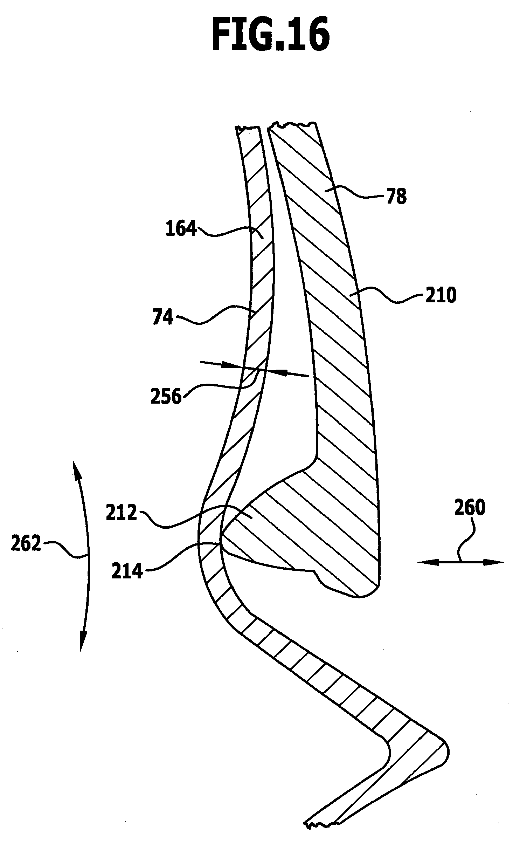

In Verbindung mit den Figuren 15 und 16 wird nachfolgend die Funktionsweise der Rückhalteelemente 212, insbesondere das Zusammenwirken mit dem Dichtelement 74, näher erläutert.

Wird, wie bereits oben beschrieben, ein Instrument, oder wie beispielsweise in Figur 11 dargestellt, der Obturator 22, von proximal her kommend in das Dichtelement 74 eingeführt, so werden auch die Rückhalteelemente 212 mit ihren Spitzen 214 in die Wand 164 des Dichtelements 74 eingedrückt. Hierfür ist es günstig, wenn eine Höhe 254 der Rückhalteelemente bezogen auf die Außenseite 210 einen Wert im Bereich von etwa 0,3 mm bis etwa 1,5 mm und eine Dicke 256 der Wand 164 einen Wert in einem Bereich von etwa 0,2 mm bis etwa 0,8 mm aufweist. Damit die Spitze 214 das Dichtelement 74 nicht beschädigen kann, insbesondere nicht durchstoßen, ist beträgt ein Radius der vorzugsweise halbkugeligen Spitze 214 des Rückhalteelements 212 etwa 0,1 mm bis etwa 0,6 mm. Die Spitze 214 wird, wie in Figur 15 dargestellt, vorzugsweise vom Dichtelement 74 umschlossen und steht mit diesem flächig in Kontakt, wodurch aufgrund einer Bewegung des Schutzelements 202, insbesondere in einer Richtung 260 quer oder im Wesentlichen quer zur Längsachse 12, eine Relativbewegung in einer hierzu senkrechten Richtung 262, das heißt parallel oder im Wesentlichen parallel zur Längsachse 12, vermieden wird. Dieser gewünschte Effekt wird besonders optimal erreicht, wenn das Dichtelement aus einem Kunststoff, insbesondere aus einem Elastomer hergestellt ist oder ein Elastomer enthält, wobei es sich zum Beispiel um Silikon oder Polyisopren handeln kann. Die gewünschte Verformung des Dichtelements, das heißt das mindestens teilweise Umfangen des Rückhalteelements 212, kann weiter verbessert werden, wenn das Material, aus dem das Dichtelement hergestellt ist, einen Shore A-Härte in einem Bereich von etwa 20 bis etwa 60 aufweist.

Die durch die Rückhalteelemente 212 verursachte Auswölbung 218 der Wand 164 ermöglicht ein Verhaken Rückhalteelemente 212 in der Wand 164, so dass die Rückhalteelemente 212 und das Dichtelement 74 quasi miteinander in Eingriff stehen. Durch das Verhaken der Rückhalteelemente 212 in der Wand 164 wird eine Relativbewegung der distalen Enden 206 der langen Schutzelemente 202 relativ zum Dichtelement 74 praktisch verhindert.

Ist das Dichtelement 74 insbesondere zu dick und zu wenig elastisch beziehungsweise zu steif oder zu hart, kann die Spitze 214 nicht die gewünschte Auswölbung 218 des Dichtelements 214 bewirken. Es kommt also gerade nicht zu der mindestens teilweisen Umschließung des Rückhalteelements 212 durch das Dichtelement 74, so dass diese nicht miteinander in Eingriff stehen, wie in Figur 16 dargestellt. Dadurch können das Dichtelement 74 und die Schutzvorrichtung 78 im Bereich der Rückhalteelemente 212 relativ zueinander bewegt werden, was eine unerwünschte Fehlfunktion des Trokarsystems bedeutet.

## Patentansprüche

1. Chirurgische Schutzvorrichtung (78) für ein chirurgisches, eine erweiterbare Einführöffnung (176) aufweisendes Dichtelement (74) eines chirurgischen, einen Trokar (14, 22) umfassenden Abdichtungssystems (10), welche Schutzvorrichtung (78) einen am Trokar (14, 22) oder an einem Teil (74) desselben anordenbaren, ringförmigen oder im Wesentlichen ringförmigen und eine Durchbrechung (192) definierenden Grundkörper (190) mit mehreren in Umfangsrichtung angeordneten und parallel oder auf eine Längsachse (12) der Schutzvorrichtung (78) hin weisenden Schutzelementen (200, 202) umfasst, welche Schutzelemente (200, 202) im Wesentlichen in distaler Richtung weisende freie Enden (204, 206) aufweisen, wobei mindestens ein Teil der Schutzelemente (202) auf einer Außenseite (210) mindestens ein Rückhalteelement (212) zum in Eingriff Bringen mit dem Dichtelement (74) aufweist, **dadurch gekennzeichnet, dass** das mindestens eine Rückhalteelement (212) an einem freien Ende (206) oder im Bereich eines freien Endes (206) eines Schutzelements (202) angeordnet ist und dass es eine vom Schutzelement (202) weg weisende Spitze (214) und eine Form aufweist, welche ein Verhaken mit einem elastischen Dichtelement (74) ermöglicht.

2. Chirurgische Schutzvorrichtung nach Anspruch 1, **dadurch gekennzeichnet, dass** das mindestens eine Rückhalteelement (212) in Form eines vom Schutzelement (202) abstehenden Rückhaltevorsprungs (212) ausgebildet ist.

3. Chirurgische Schutzvorrichtung nach Anspruch 1 oder 2, **dadurch gekennzeichnet, dass** die Spitze (214) kegelförmig oder kugelförmig ist.

4. Chirurgische Schutzvorrichtung nach Anspruch 1 oder 2, **dadurch gekennzeichnet, dass** die Spitze (214) einen Radius in einem Bereich von etwa 0,1 mm bis etwa 0,6 mm aufweist.

5. Chirurgische Schutzvorrichtung nach einem der voranstehenden Ansprüche, **gekennzeichnet durch** eine am Grundkörper (190) angeordnete Verbindungseinrichtung (188, 220) zum Verbinden der Schutzvorrichtung mit dem chirurgischen Dichtelement (74) oder dem chirurgischen Abdichtungssystem (10).

6. Chirurgische Schutzvorrichtung nach einem der voranstehenden Ansprüche, **dadurch gekennzeichnet, dass** in einer Grundstellung der Schutzvorrichtung (78) benachbarte Schutzelemente (200, 202) sich gegenseitig teilweise überdecken.

7. Chirurgische Schutzvorrichtung nach einem der voranstehenden Ansprüche, **dadurch gekennzeichnet, dass** in einer Grundstellung der Schutzvorrichtung (78) die Rückhalteelemente (212) tragenden Schutzelemente (202) die Schutzelemente (200) ohne Rückhalteelemente (212) außen mindestens teilweise überdecken.

8. Chirurgische Schutzvorrichtung nach einem der voranstehenden Ansprüche, **dadurch gekennzeichnet, dass** die Schutzelemente (200, 202) in sich flexibel sind.

9. Chirurgisches Abdichtungssystem (10) umfassend einen Trokar (14, 22) mit einer Trokarhülse (14), ein an der Trokarhülse (14) gehaltenes chirurgisches, eine erweiterbare Einführöffnung (176) aufweisendes Dichtelement (74) zum Abdichten der Einführöffnung (176) beim Einführen eines chirurgischen Instruments (22) und eine chirurgische Schutzvorrichtung (78) für das Dichtelement (74), welche Schutzvorrichtung (78) einen am Trokar (14, 22) oder am Dichtelement (74) anordenbaren, ringförmigen oder im Wesentlichen ringförmigen und eine Durchbrechung (192) definierenden Grundkörper (190) mit mehreren in Umfangsrichtung angeordneten und parallel oder auf eine Längsachse (12) der Schutzvorrichtung (78) hin weisenden Schutzelementen (200, 202) umfasst, welche Schutzelemente (200, 202) im Wesentlichen in distaler Richtung weisende freie Enden (204, 206) aufweisen, wobei mindestens ein Teil der Schutzelemente (200, 202) auf einer Außenseite (210) mindestens ein Rückhalteelement (212) zum In Eingriff Bringen mit dem Dichtelement (74) aufweisen, **dadurch gekennzeichnet, dass** das mindestens eine Rückhalteelement (212) an einem freien Ende (206) oder im Bereich eines freien Endes (206) eines Schutzelements (202) angeordnet ist und dass es eine vom Schutzelement (202) weg weisende Spitze (214) und eine Form aufweist, welche ein Verhaken mit einem elastischen Dichtelement (74) ermöglicht.

10. Chirurgisches Abdichtungssystem nach Anspruch 9, **gekennzeichnet durch** eine Schutzvorrichtung (78) nach einem der Ansprüche 2 bis 9.

11. Chirurgisches Abdichtungssystem nach Anspruch 9 oder 10, **dadurch gekennzeichnet, dass** das Dichtelement (74) eine ringförmig geschlossene, flexible Wand (164) umfasst, dass die Wand (164) einen ersten und einen zweiten, jeweils in sich geschlossenen Rand (148, 166) aufweist und dass der erste Rand (166) die Öffnung (176) begrenzt und
dass die Wand (164) wellenförmig faltbar und in einer Dichtstellung derart wellenförmig ohne Knicke mit in Richtung auf den ersten Rand (166) hin verlaufenden Faltlinien gefaltet ist, dass der erste Rand (166) eine Wellenlinie (168) definiert, welche vollständig auf einer Zylinderfläche liegt.

12. Chirurgisches Abdichtungssystem nach einem der Ansprüche 9 bis 11, **dadurch gekennzeichnet, dass** die Wellenlinie (168) oberhalb einer senkrecht zur Längsachse (12) verlaufenden Öffnungsebene (146) der Öffnung (176) Wellenberge (170) und unterhalb der Öffnungsebene (146) Wellentäler (172) aufweist.

13. Chirurgisches Abdichtungssystem nach Anspruch 12, **dadurch gekennzeichnet, dass** die freien Enden (206) der Rückhalteelemente (212) tragenden Schutzelemente (202) zwischen die Wellenberge (170) und/oder Wellentäler (172) eingreifen.

14. Chirurgisches Abdichtungssystem nach einem der Ansprüche 9 bis 13, **dadurch gekennzeichnet, dass** die Schutzvorrichtung (78) mit dem Dichtelement (74) lösbar verbindbar ist.

15. Chirurgisches Abdichtungssystem nach einem der Ansprüche 12 bis 14, **dadurch gekennzeichnet, dass** das Dichtelement (74) zu den Verbindungselementen (188) der Verbindungsvorrichtung (220) korrespondierende Verbindungsglieder (186) aufweist.

## Claims

1. Surgical protection device (78) for a surgical sealing element (74) of a surgical sealing system (10) comprising a trocar (14, 22), said sealing element having an insertion opening (176) adapted to be widened, said protection device (78) comprising a base member (190) adapted to be arranged on the trocar (14, 22) or on a part (74) thereof, being in a ring shape or essentially in a ring shape and defining an opening (192) and comprising several protection elements (200, 202) arranged in circumferential direction and pointing parallel or towards a longitudinal axis (12) of the protection device (78), said protection elements (200, 202) having free ends (204, 206) pointing essentially in a distal direction, wherein at least some of the protection elements (202) have at least one retaining element (212) on an outer side (210) for engagement with the sealing element (74), **characterized in that** the at least one retaining element (212) is arranged at a free end (206) or in the area of a free end (206) of a protection element (202) and **in that** it has a tip (214) pointing away from the protection element (202) and a shape making it possible for them to become caught in an elastic sealing element (74).

2. Surgical protection device according to claim 1, **characterized in that** the at least one retaining element (212) is designed in the form of a retaining projection (212) protruding from the protection element (202).

3. Surgical protection device according to claim 1 or 2, **characterized in that** the tip (214) is of a conical or spherical shape.

4. Surgical protection device according to claim 1 or 2, **characterized in that** the tip (214) has a radius in a range of approximately 0.1 mm to approximately 0.6 mm.

5. Surgical protection device according to any one of the preceding claims, **characterized by** a connecting device (188, 220) arranged on the base member (190) for connecting the protection device to the surgical sealing element (74) or the surgical sealing system (10).

6. Surgical protection device according to any one of the preceding claims, **characterized in that** adjacent protection elements (200, 202) partially cover one another in a basic position of the protection device (78).

7. Surgical protection device according to any one of the preceding claims, **characterized in that** the protection elements (202) bearing retaining elements (212) cover the protection elements (200) without retaining elements (212) at least partially on the outside in a basic position of the protection device (78).

8. Surgical protection device according to any one of the preceding claims, **characterized in that** the protection elements (200, 202) are flexible in themselves.

9. Surgical sealing system (10) comprising a trocar (14, 22) with a trocar sleeve (14), a surgical sealing element (74) having an insertion opening (176) adapted to be widened and being held on the trocar sleeve (14) for sealing the insertion opening (176) during insertion of a surgical instrument (22) and a surgical protection device (78) for the sealing element (74), said protection device (78) comprising a base member (190) adapted to be arranged on the trocar (14, 22) or on the sealing element (74), being in a ring shape or essentially in a ring shape and defining an opening (192) and having several protection elements (200, 202) arranged in circumferential direction and pointing parallel or towards a longitudinal axis (12) of the protection device (78), said protection elements (200, 202) having free ends (204, 206) pointing essentially in a distal direction, wherein at least some of the protection elements (200, 202) have at least one retaining element (212) on an outer side (210) for engagement with the sealing element (74), **characterized in that** the at least one retaining element (212) is arranged at a free end (206) or in the area of a free end (206) of a protection element (202) and **in that** it has a tip (214) pointing away from the protection element (202) and a shape making it possible for them to become caught in an elastic sealing element (74).

10. Surgical sealing system according to claim 9, **characterized by** a protection device (78) according to any one of claims 2 to 9.

11. Surgical sealing system according to claim 9 or 10, **characterized in that** the sealing element (74) comprises a flexible wall (164) closed in a ring shape, **in that** the wall (164) has a first and a second edge (148, 166) each closed upon itself and **in that** the first edge (166) delimits the opening (176) and **in that** the wall (164) is adapted to be folded in a wave-like manner and in a sealing position is folded in a wave-like manner without any kinks with fold lines extending in a direction towards the first edge (166) in such a manner that the first edge (166) defines a wave line (168) located entirely on a cylindrical surface.

12. Surgical sealing system according to any one of claims 9 to 11, **characterized in that** the wave line (168) has wave peaks (170) above an opening plane (146) of the opening (176) extending at right angles to the longitudinal axis (12) and wave troughs (172) below the plane of opening (146).

13. Surgical sealing system according to claim 12, **characterized in that** the free ends (206) of the protection elements (202) bearing retaining elements (212) engage between the wave peaks (170) and/or wave troughs (172).

14. Surgical sealing system according to any one of claims 9 to 13, **characterized in that** the protection device (78) is adapted to be connected detachably to the sealing element (74).

15. Surgical sealing system according to any one of claims 12 to 14, **characterized in that** the sealing element (74) has connecting members (186) corresponding to the connecting elements (188) of the connecting device (220).

## Revendications

1. Dispositif de protection chirurgical (78) pour un élément d'étanchéité (74), qui présente une ouverture d'introduction (176) pouvant être évasée, et qui fait partie d'un système d'étanchéité chirurgical (10) comprenant un trocart (14, 22), ledit dispositif de protection (78) comprenant un corps de base (190), qui peut être agencé sur le trocart (14, 22) ou une partie (74) de celui-ci, qui est de forme annuaire ou sensiblement de forme annulaire et définit un passage (192), et qui comporte plusieurs éléments de protection (200, 202) agencés en direction périphérique, parallèles ou dirigés vers un axe longitudinal (12) du dispositif de protection (78), lesdits éléments de protection (200, 202) présentant des extrémités libres (204, 206) dirigées sensiblement en direction distale, au moins une partie des éléments de protection (202) comportant sur un côté extérieur (210), au moins un élément de retenue (212) destiné à être amené en prise avec l'élément d'étanchéité (74), **caractérisé en ce que** ledit au moins un élément de retenue (212) est agencé à une extrémité libre (206) ou dans la zone d'une extrémité libre (206) d'un élément de protection (202), et **en ce qu'**il présente une pointe (214) orientée de manière à s'éloigner de l'élément de protection (202), et une forme permettant de s'accrocher à un élément d'étanchéité (74) élastique.

2. Dispositif de protection chirurgical selon la revendication 1, **caractérisé en ce que** ledit au moins un élément de retenue (212) est réalisé sous la forme d'une protubérance de retenue (212) faisant saillie de l'élément de protection (202).

3. Dispositif de protection chirurgical selon la revendication 1 ou la revendication 2, **caractérisé en ce que** la pointe (214) est de forme conique ou de forme sphérique.

4. Dispositif de protection chirurgical selon la revendication 1 ou la revendication 2, **caractérisé en ce que** la pointe (214) présente un rayon situé dans une plage d'environ 0,1 mm à environ 0,6 mm.

5. Dispositif de protection chirurgical selon l'une des revendications précédentes, **caractérisé par** un dispositif de liaison (188, 220) agencé sur le corps de base (190) et destiné à relier le dispositif de protection à l'élément d'étanchéité chirurgical (74) ou au système d'étanchéité chirurgical (10).

6. Dispositif de protection chirurgical selon l'une des revendications précédentes, **caractérisé en ce que** dans une position initiale ou de base du dispositif de protection (78), des éléments de protection voisins (200, 202) se recouvrent partiellement de manière réciproque.

7. Dispositif de protection chirurgical selon l'une des revendications précédentes, **caractérisé en ce que** dans une position initiale ou de base du dispositif de protection (78), les éléments de protection (202) portant les éléments de retenue (212) recouvrent au moins partiellement, à l'extérieur, les éléments de protection (200) sans éléments de retenue (212).

8. Dispositif de protection chirurgical selon l'une des revendications précédentes, **caractérisé en ce que** les éléments de protection (200, 202) sont flexibles en soi.

9. Système d'étanchéité chirurgical (10) comprenant un trocart (14, 22) avec un tube de trocart (14), un élément d'étanchéité chirurgical (74), qui est maintenu sur le tube de trocart (14) et présente une ouverture d'introduction (176) pouvant être évasée, et qui est destiné à assurer l'étanchéité de l'ouverture d'introduction (176) lors de l'introduction d'un instrument chirurgical (22), et un dispositif de protection chirurgical (78) pour l'élément d'étanchéité (74), ledit dispositif de protection (78) comprenant un corps de base (190), qui peut être agencé sur le trocart (14, 22) ou sur l'élément d'étanchéité (74), qui est de forme annuaire ou sensiblement de forme annulaire et définit un passage (192), et qui comporte plusieurs éléments de protection (200, 202) agencés en direction périphérique, parallèles ou dirigés vers un axe longitudinal (12) du dispositif de protection (78), lesdits éléments de protection (200, 202) présentant des extrémités libres (204, 206) dirigées sensiblement en direction distale, au moins une partie des éléments de protection (200, 202) comportant sur un côté extérieur (210), au moins un élément de retenue (212) destiné à être amené en prise avec l'élément d'étanchéité (74), **caractérisé en ce que** ledit au moins un élément de retenue (212) est agencé à une extrémité libre (206) ou dans la zone d'une extrémité libre (206) d'un élément de protection (202), et **en ce qu'**il présente une pointe (214) orientée de manière à s'éloigner de l'élément de protection (202), et une forme permettant de s'accrocher à un élément d'étanchéité (74) élastique.

10. Système d'étanchéité chirurgical selon la revendication 9, **caractérisé par** un dispositif de protection (78) selon l'une des revendications 2 à 9.

11. Système d'étanchéité chirurgical selon la revendication 9 ou la revendication 10, **caractérisé en ce que** l'élément d'étanchéité (74) comprend une paroi flexible (164) de forme annulaire fermée, **en ce que** la paroi (164) présente un premier et un deuxième bord (148, 166) respectivement fermé en soi, et **en ce que** le premier bord (166) délimite l'ouverture (176), et **en ce que** la paroi (164) peut être plissée sous forme ondulée et, dans une position d'étanchéité, est plissée de façon ondulée, sans plis marqués, avec des lignes de plissage s'étendant en direction du premier bord (166) de manière telle, que le premier bord (166) définisse une ligne d'ondulation (168), qui se situe en totalité sur une surface de cylindre.

12. Système d'étanchéité chirurgical selon l'une des revendications 9 à 11, **caractérisé en ce que** la ligne d'ondulation (168) présente, au-dessus d'un plan d'ouverture (146) de l'ouverture (176), qui s'étend perpendiculairement à l'axe longitudinal (12), des sommets d'onde (170), et en-dessous du plan d'ouverture (146), des creux d'onde (172).

13. Système d'étanchéité chirurgical selon la revendication 12, **caractérisé en ce que** les extrémités libres (206) des éléments de protection (202) portant des éléments de retenue (212), s'engagent entre les sommets d'onde (170) et/ou les creux d'onde (172).

14. Système d'étanchéité chirurgical selon l'une des revendications 9 à 13, **caractérisé en ce que** le dispositif de protection (78) peut être relié de manière amovible avec l'élément d'étanchéité (74).

15. Système d'étanchéité chirurgical selon l'une des revendications 12 à 14, **caractérisé en ce que** l'élément d'étanchéité (74) comporte des organes de liaison (186) correspondant aux éléments de liaison (188) du dispositif de liaison (220).
